# EUROPEAN PATENT APPLICATION

(11) **EP 3 244 310 A1**
(43) Date of publication of application: **15.11.2017**
(21) Application number: 17170494.3
(22) Date of filing: 10.05.2017
(51) Int. Cl.: G06F 9/50, G06F 1/32, G06F 19/00, H04L 29/06, G06F 9/48, G06F 9/54

(54) **A COMPANION APP TO COOPERATE WITH AN INTEGRATED TRACKER APP**

(30) Priority: 11.05.2016 US 201662334959 P; 09.05.2017 US 201715590392
(71) Applicant: Fitbit, Inc., San Francisco, CA 94105 (US)
(72) Inventor: SARLANDIE DE LA ROBERTIE, Thomas, San Mateo, CA 94401 (US); CASE, Ryan James, Waterloo, Ontario N2L 2Y6 (CA); MURRAY, Brad, Kitchener, Ontario N2G 0B5 (CA); JACKWERTH, Marcel, 20099 Hamburg (DE); REBAUD, Sylvain, San Francisco, CA 94127 (US); TROITINO, Daniel Rodriguez, Menlo Park, CA 94025 (US)
(74) Representative: WP Thompson

(57) **Abstract**

A mobile app (120) generates and manages companion apps (115, 315) in a memory (118, 220, 830) of the mobile computing device. The companion apps (115, 315) cooperate with one or more integrated tracker apps (216) in a memory (118, 220, 830) of a wearable device to provide a combined functionality to the wearable device. A companion app (115) communicates with an integrated tracker app (216) to provide at least extended capabilities for the integrated tracker app (216) including adding connectivity to the Internet to access On-line services and gather data on behalf of the integrated tracker app (216) via the integrated tracking app (216) submitting a request to an application programming interface (840, 850) for the companion app (115). The mobile app (120) coordinates activities between i) multiples instances of the companion app (115) loaded in the memory (118, 220, 830) of the mobile computing device and ii) the integrated tracker apps (216) running in the memory (118, 220, 830) of the wearable device.

## Description

### CROSS-REFERENCE

This application claims priority to U.S. Provisional Patent Application No. US 62/334959, filed May 11, 2016, titled "METHODS OF COMPANION ELECTRONIC DEVICES AND SYSTEMS," the disclosure of which is hereby incorporated herein by reference in its entirety.

### FIELD

The design generally relates to wearable electronics devices with greater capabilities and functionality because one or more companion apps cooperate with one or more integrated tracker apps to provide a combined functionality to the wearable device.

### BACKGROUND

Typically, a wearable electronic device can be used as a passive device, such as a watch to provide the time and merely track a total amount of steps a user of the device has taken that day. The wearable electronic device generally may be limited via computing power, battery life, display screen size, etc. on the type of things that are possible with the wearable electronic device.

### SUMMARY

In general, various methods and apparatuses are discussed for a wearable device with greater capabilities and functionality because one or more companion apps cooperate with one or more integrated tracker apps to provide a combined functionality to the wearable device.

In accordance with a first aspect of the invention, there is provided a non-transitory machine-readable medium configured to store instructions and data, which when executed by one or more processors on a mobile computing device, causes the following operations, comprising:
a mobile app generating and managing one or more companion apps in a memory of the mobile computing device, where the one or more companion apps in the memory of the mobile computing device cooperate with one or more integrated tracker apps in a memory of a wearable device to provide a combined functionality to the wearable device, where a first companion app is in communication with a first integrated tracker app to provide at least extended capabilities for the first integrated tracker app including adding connectivity to an Internet to access On-line services and gather data on behalf of the first integrated tracker app via the first integrated tracking app submitting a request to a fetch application programming interface for the first companion app;
executing instructions for the first integrated tracker app with a first processor in the wearable device, and executing instructions for the first companion app with a second processor in the mobile computing device;
where the mobile app coordinates activities between i) multiples instances of the companion app loaded in the memory of the mobile computing device and ii) the one or more integrated tracker apps running in the memory of the wearable device;
where the first integrated tracker app at least consumes less battery power and consumes less computing cycles from the first processor on the wearable device than if the first integrated tracker app performed all of the combined functionality given to the wearable device from the first integrated tracker app cooperating with the first companion app; and
where the first integrated tracker app utilizes the mobile computing device's capabilities via the first companion app as opposed to interacting directly with an operating system on the mobile computing device.

The non-transitory machine-readable medium of the invention may further comprise:
triggering the companion app to launch in the memory on the mobile computing device in response a user launching the first integrated tracker app on the wearable device;
running the first companion app in the memory of the mobile computing device while the wearable device is not running an executable file of the first integrated tracker app in order to allow the first companion app to extend capabilities of the first integrated tracker app by interacting with the Internet web services on behalf of the first integrated tracker app while the wearable device is not running the first integrated tracker app; and
further extending a computational and memory storage capabilities available to the first integrated tracker app by the first companion app performing a distributed workflow using the second processor and memory of the mobile computing device on behalf of the first integrated tracker app.

The non-transitory machine-readable medium of the invention may further comprise:
continuing to collect tracked data and give the user of the wearable device feedback on an activity that user is engaged in, via any of a display, a vibration, a turning on or off of lights, and an emitting of a sound, when the first integrated tracker app resident on the wearable device is disconnected with the first companion app on the mobile computing device; and
periodically, checking to see if a wireless communication circuit of the mobile computing device is within wireless range to establish a connection between a wireless communication circuit of the wearable device and the wireless communication circuit of the mobile computing device, where when the companion app on the mobile computing device is loaded in memory and within range, the first integrated tracker app will migrate all of the tracked data when the first companion app and first integrated tracker app pair together and then synchronize the tracked data from the wearable device into the mobile computing device.

The non-transitory machine-readable medium of the invention may further comprise:
configuring settings' updates for the first integrated tracker app on the wearable device i) through a user interface of the first companion app displayed on a display screen of the mobile computing device and ii) through use of a diversity of user input mechanisms of the mobile computing device, including any of a combination of a keyboard input, a voice recognition input, or a touch screen input for the mobile computing device, which allows a user of the first integrated tracker app on the wearable device to take advantage of a bigger display screen and a diverse amount of user input mechanisms of the mobile computing device, via the user interface of the first companion app, to make settings changes that are then conveyed and implemented on the first integrated tracker app on the wearable device.

The non-transitory machine-readable medium of the invention may further comprise:
where the mobile app is configured to run multiple instances of the companion app concurrently in the memory of the mobile computing device and form a pool of instances of companion apps that are running in the memory of the mobile computing device, where all of the multiple instances of the companion app in the pool are related to each other in that one companion app can be promoted over another companion app, where the first companion app is associated with the first integrated tracker app on the wearable device, a second companion app is associated with a second integrated tracker app on the wearable device.

In accordance with a second aspect of the invention, there is provided an apparatus, comprising:
a mobile app resident in a memory of a mobile computing device having multiple components including one or more event handers, a state controller, a scheduler, and an application programming interface; where the mobile app is configured to generate and manage one or more companion apps in the memory of the mobile computing device, where the one or more companion apps in the memory of the mobile computing device are coded to cooperate with one or more integrated tracker apps in a memory of the wearable device to provide a combined functionality to the wearable device;
where a first companion app is coded to be in communication with a first integrated tracker app to provide functionality selected from a group consisting of i) perform data collection by the first companion app on behalf of the first integrated tracker app, ii) facilitate settings updates for the first integrated tracker app on the wearable device configured through a user interface of the first companion app displayed on a display screen of the mobile computing device, and iii) extend capabilities of the first integrated tracker app;
where the one or more integrated tracker apps are resident in the memory of the wearable device and are configured to wirelessly pair through a wireless communication circuit of the wearable device to the one or more companion apps loaded in the memory of the mobile computing device, where the first integrated tracker app on the wearable device is configured to submit requests for the one or more event handlers through the application programming interface of the mobile app, where the instructions for the first integrated tracker app are executed by a first processor of the wearable device and instructions for the first companion app are executed by a second processor of the mobile computing device;
where the first companion app is configured to run in the memory of the mobile computing device while the wearable device is not running an executable file of the first integrated tracker app in order to allow the first companion app to perform functions selected from a group consisting of i) pre-fetch data on behalf of the first integrated tracker app, ii) record settings updates to the first integrated tracker app on the wearable device that are configured through the user interface of the companion app displayed on the display screen of the mobile computing device, iii) extend a computational and memory storage capabilities available to the first integrated tracker app via a distributed workflow between the first integrated tracker app and the companion app, iv) extend capabilities of the first integrated tracker app by adding connectivity to an Internet to access On-line services and gather data on behalf of the first integrated tracker app via the first integrated tracking app submitting a request to the application programming interface for the first companion app, and v) obtain sensor data from one or more sensors in the mobile computing device on behalf of the first integrated tracker app;
wherein the state controller of the mobile app is configured to regulate two main operational states of the first companion app i) loaded and ii) unloading, where the scheduler is configured to cooperate with the state controller for the companion app to coordinate activities between i) multiples instances of the companion app loaded in the memory of the mobile computing device and ii) the one or more integrated tracker apps running in the memory of the wearable device; and
where the first integrated tracker app occupies less memory space, consumes less battery power, and consumes less computing cycles from the first processor on the wearable device than if the first integrated tracker app performed all of the combined functionality given to the wearable device from the first integrated tracker app cooperating with the first companion app.

In the apparatus of the present invention, the state controller for the companion app may be configured to trigger an operational state of launched in the memory on the mobile computing device for various detected situations selected from a group consisting of
i) in response a user launching the first integrated tracker app on the wearable device, then a first message is communicated to the state controller to launch the first companion app,
ii) when the first companion app has requested to be woken up at periodic time intervals because the first integrated tracker app and first companion have not been in communication for a threshold amount of time, then the first companion app is launched in order to pre-fetch data that will be used by the first tracker app once it is launched on the wearable device, as well as
iii) when the monitoring routine of the companion app communicates the second message that a significant location change has happened for the wearable device, where the significant location change is equal to or above a location change threshold set by the companion app.

In the apparatus of the present invention, the first companion app may be configured to provide integrated functionality on behalf of the first integrated app for all three of i) data collect by the first companion app on behalf of the first integrated tracker app, ii) provide settings updates for the first integrated tracker app on the wearable device configured through the user interface of the first companion app displayed on the display screen of the mobile computing device, as well as iii) extend capabilities of the first integrated tracker app;
where the state controller for the companion app is configured to trigger an operational state of launched in the memory on the mobile computing device for all three of
i) in response the user launching the first integrated tracker app on the wearable device, then the first message is communicated to the state controller to launch the first companion app,
ii) when the first companion app has requested to be woken up at periodic time intervals because the tracker app and companion have not been in communication for a threshold amount of time, then the first companion app is launched in order to pre-fetch data that will be used by the first tracker app once it is launched on the wearable device, as well as
iii) when the monitoring routine of the companion app communicates the second message that the significant location change has happened for the wearable device, where the significant location change is equal to or above the location change threshold set by the companion app; and
where the mobile app is not part of an operating system of the mobile computing device but rather a standalone mobile app; where the wearable device is a fitness tracker and the mobile computing device is a smart phone.

In the apparatus of the present invention, the state controller may be configured to transition the first companion app into and out of sub states of the main operational state of unloading in the memory, where the sub states include at least i) Dead - awaiting launch of the first companion app via the state controller; and ii) Dying - the state controller is coded to expect a communication from the first integrated tracker app that the first integrated tracker app will not send any more events during this life cycle of the first companion app and accordingly the first companion app should be unloaded from the memory of the mobile computing device when all of the event handlers for the first companion app return their results back to the first integrated tracker app.

In the apparatus of the present invention, the state controller may be configured to transition the first companion app into and out of sub states of the main operational state of loaded in the memory, where the sub states include at least i) Queued - the state controller has triggered the first companion app to be executed but it is awaiting an available slot by the scheduler; ii) Launched - the first companion app is being executed and run in the memory of the mobile computing device, iii) Promoted - the first companion app is running concurrently with other companion apps and has priority for scheduling and servicing over one or more of the companion apps, and in addition the first companion app will keep this sub state context running until an event triggers its demotion, iv) Demoted - the first companion app is handling events but no corresponding first integrated tracker app is currently running in the memory of wearable device.

In the apparatus of the present invention, the scheduler of the mobile app may be configured to run multiple instances of the companion app concurrently in the memory of the mobile computing device and form a pool of instances of companion apps that are running in the memory of the mobile computing device, where all of the multiple instances of the companion app in the pool are related to each other in that one companion app can be promoted for scheduling and servicing over another companion app.

In the apparatus of the present invention, the scheduler of the mobile app may be configured to limit an amount of instances of companion apps running concurrently in the pool to a threshold set amount, where when launching another instance of the companion app would violate the threshold set amount of the pool, then the scheduler will put a next instance of the companion app into a priority queue, where in the priority queue, promoted companion apps have priority over all other companion apps in a different sub state including a demoted companion app.

In the apparatus of the present invention, the first companion app may be configured to cooperate with and leverage an authorization proxy for an existing web service to obtain an authentication token to access the user of the wearable device's protected resources at a third party service without asking the user of the wearable device to type their login or password on the wearable device.

In the apparatus of the present invention,
the first integrated tracker app resident on the wearable device may be configured to collect the user's tracked data, where the first integrated tracker app is coded that when disconnected with the first companion app on the mobile computing device, then the first integrated tracker app is configured to continue to collect information and give the user of the wearable device feedback on an activity that user is engaged in, via any of a display, a vibration, a turning on or off of lights, and an emitting of a sound,
a tracker operating system of the wearable device may be configured to periodically check to see if the wireless communication circuit of the mobile computing device is within wireless range to establish a connection between the wireless communication circuit of the wearable device and the wireless communication circuit of the mobile computing device, and
the first companion app on the mobile computing device may be loaded in the memory and within range to establish the connection, the first integrated tracker app is configured to migrate all of the tracked data when the two applications communicate with each other, and then the first companion app is configured to synchronize the tracked data from the wearable device into the mobile computing device.

In the apparatus of the present invention, the state controller may be configured to trigger the sub state of launched for the first companion app on the mobile computing device when the first tracker app has been launched on the wearable device by the user so that the first companion app can perform any of i) pre-fetch data from an Internet source and ii) pre-fetch sensor data from sensors on the mobile computing device; and thereby, minimize a time period it takes for the first tracker app to display up to date information on a display screen of the wearable device when the user launches the first tracker app on the wearable device.

In the apparatus of the present invention, the first companion app on the mobile device may be configured to receive a timely event notice from the mobile app on the mobile computing device before the state controller for the first companion app transitions the first companion app to a dead operational state in order to improve a functioning of the mobile computing device in at least two ways i) so that the first companion app is configured to save state in response to receiving the timely event notice; and thus, does not have to continuously save state all the time, as well as ii) the first companion app is configured to make network requests merely when the first companion app is likely to have enough time to complete this task prior to be transitioned to the dead operational state, where the timely event notice allows the first companion app to minimize 1) a need for CPU cycles from the second processer and 2) a cumulative amount of network requests made by the first companion app on the mobile computing device.

In accordance with a third aspect of the invention, a method for one or more tracker apps in a memory of wearable device to cooperate with one or more companion apps in a memory of a mobile computing device, comprises:
a mobile app generating and managing the one or more companion apps in the memory of the mobile computing device, where the one or more companion apps in the memory of the mobile computing device cooperate with the one or more integrated tracker apps in the memory of the wearable device to provide a combined functionality to the wearable device, where a first companion app is in communication with a first integrated tracker app to provide at least extended capabilities for the first integrated tracker app including adding connectivity to an Internet to access On-line services and gather data on behalf of the first integrated tracker app via the first integrated tracking app submitting a request to a fetch application programming interface for the first companion app;
executing instructions for the first integrated tracker apps with a first processor in the wearable device, and executing instructions for the first companion app with a second processor in the mobile computing device;
where the mobile app coordinates activities between i) multiples instances of the companion app loaded in the memory of the mobile computing device and ii) the one or more integrated tracker apps running in the memory of the wearable device;
where the first integrated tracker app at least consumes less battery power and consumes less computing cycles from the first processor on the wearable device than if the first integrated tracker app performed all of the combined functionality given to the wearable device from the first integrated tracker app cooperating with the first companion app; and
where the first integrated tracker app utilizes the mobile computing device's capabilities via the first companion app as opposed to interacting directly with an operating system on the mobile computing device.

The method of the invention may further comprise:
triggering the companion app to launch in the memory on the mobile computing device in response a user launching the first integrated tracker app on the wearable device;
running the first companion app in the memory of the mobile computing device while the wearable device is not running an executable file of the first integrated tracker app in order to allow the companion app to extend capabilities of the first integrated tracker app by interacting with the Internet web services on behalf of the first integrated tracker app while the wearable device is not running the first integrated tracker app; and
further extending a computational and memory storage capabilities available to the first integrated tracker app by the first companion app performing a distributed workflow using the second processor and memory of the mobile computing device on behalf of the first integrated tracker app.

The method of the invention may further comprise:
continuing to collect tracked data and give the user of the wearable device feedback on an activity that user is engaged in, via any of a display, a vibration, a turning on or off of lights, and an emitting of a sound, when the first integrated tracker app resident on the wearable device is disconnected with the first companion app on the mobile computing device; and
periodically, checking to see if a wireless communication circuit of the mobile computing device is within wireless range to establish a connection between a wireless communication circuit of the wearable device and the wireless communication circuit of the mobile computing device, where when the first companion app on the mobile computing device is loaded in memory and within range, the first integrated tracker app will migrate all of the tracked data when the companion app and first integrated tracker app pair together and then synchronize the tracked data from the wearable device into the mobile computing device.

The method of the invention may further comprise:
configuring settings' updates for the first integrated tracker app on the wearable device i) through a user interface of the first companion app displayed on a display screen of the mobile computing device and ii) through use of a diversity of user input mechanisms of the mobile computing device, including any of a combination of a keyboard input, a voice recognition input, or a touch screen input for the mobile computing device, which allows a user of the tracker app on the wearable device to take advantage of a bigger display screen and diverse user input mechanisms of the mobile computing device, via the user interface of the first companion app, to make setting changes that are then conveyed and implemented on the first tracker app on the wearable device.

In an embodiment, a mobile app generates and manages companion apps in a memory of the mobile computing device. The companion apps cooperate with one or more integrated tracker apps in a memory of a wearable device to provide a combined functionality to the wearable device. A companion app communicates with an integrated tracker app to provide at least extended capabilities for the integrated tracker app including adding connectivity to the Internet to access On-line services and gather data on behalf of the integrated tracker app via the integrated tracking app submitting a request to a fetch application programming interface for the companion app.

One or more processors in the wearable device execute instructions for the integrated tracker apps. One or more processors in the mobile computing device execute instructions for the companion apps.

The mobile app coordinates activities between i) multiples instances of the companion app loaded in the memory of the mobile computing device and ii) the integrated tracker apps running in the memory of the wearable device.

### DRAWINGS

The multiple drawings refer to the example embodiments of the design.
Figure 1 illustrates a diagram of an embodiment of a mobile app resident in a memory of a mobile computing device that generates and manages one or more companion apps.
Figure 2 illustrates a diagram of an embodiment of wearable device having one or more integrated tracker apps cooperating with the one or more companion apps.
Figure 3 illustrates a diagram of an embodiment of the mobile app that is configured to run multiple instances of the companion app concurrently in the memory of the mobile computing device and form a pool of instances of companion apps that are running in the memory of the mobile computing device and cooperating via one or more application programming interfaces with multiple integrated tracker apps.
Figure 4A illustrates a state diagram of an embodiment of a companion app's life cycle that include main operational states of i) loaded into the volatile memory, and ii) unloading out of the volatile memory of the mobile computing device.
Figure 4B illustrates a state diagram of an embodiment of a companion app's life cycle that include sub states of at least i) Dead, ii) Dying, iii) Queued, iv) Launched, v) Promoted, and vi) Demoted.
Figure 5 illustrates a diagram of an embodiment of a companion app extending capabilities of the integrated tracker app by interacting with the Internet web services on behalf of the integrated tracker app even when the wearable device is not running the integrated tracker app.
Figure 6 illustrates two or more wearable electronic devices communicating with other electronic devices on a network in accordance with some embodiments.
Figure 7 illustrates a computing system that can be part of one or more of the wearable electronic devices, one or more mobile computing devices, and server systems, in accordance with some embodiments.
Figures 8A-8D illustrate a method 800 with respect to a mobile app generating and managing one or more companion apps that cooperate with one or more integrated tracker apps to provide a combined functionality to the wearable device in accordance with some embodiments.

While the design is subject to various modifications and alternative forms, specific embodiments thereof have been shown by way of example in the drawings and will herein be described in detail. The design should be understood to not be limited to the particular forms disclosed, but on the contrary, the intention is to cover all modifications, equivalents, and alternatives falling within the spirit and scope of the design.

### DESCRIPTION

In the following description, numerous specific details are set forth, such as examples of wearable electronic devices, named components, connections, number of databases, etc., in order to provide a thorough understanding of the present design. It will be apparent; however, to one skilled in the art that the present design may be practiced without these specific details. In other instances, well known components or methods have not been described in detail but rather in a block diagram in order to avoid unnecessarily obscuring the present design. A 'first' app need not refer to specific component or embodiment. Rather a 'first' app is merely different than a 'second' app. Thus, the specific details set forth are merely exemplary. The specific details discussed in one embodiment may be reasonably implemented in another embodiment. The specific details may be varied from and still be contemplated to be within the spirit and scope of the present design.

In general, the design splits apps in multiple pieces and uses one or more companion apps in a more powerful computing device, such as a smart phone, which has more resources than a wearable device in order to delegate some of the computing needs and functionalities for the benefit of one or more apps on the wearable device. Each companion app may be coded with JavaScript. In an embodiment, a mobile app, as opposed to, for example, an operating system, generates and manages companion apps in a memory of the mobile computing device. The companion apps cooperate with one or more integrated tracker apps in a memory of a wearable device to provide a combined functionality to the wearable device. A companion app communicates with an integrated tracker app to provide at least extended capabilities for the integrated tracker app. The mobile app, as opposed to, for example, an operating system, coordinates activities between i) multiples instances of the companion app loaded in the memory of the mobile computing device and ii) the integrated tracker apps running in the memory of the wearable device.

Note, in general, an app herein includes but is not limited to i) standalone software applications, ii) software instructions and data for a standalone mobile app and/or wearable device apps, and iii) programs that provide a specific functionality but happen to be that are part of an operating system application. However, specifically the mobile app that generates and manages one or more companion apps is not part of an operating system application.

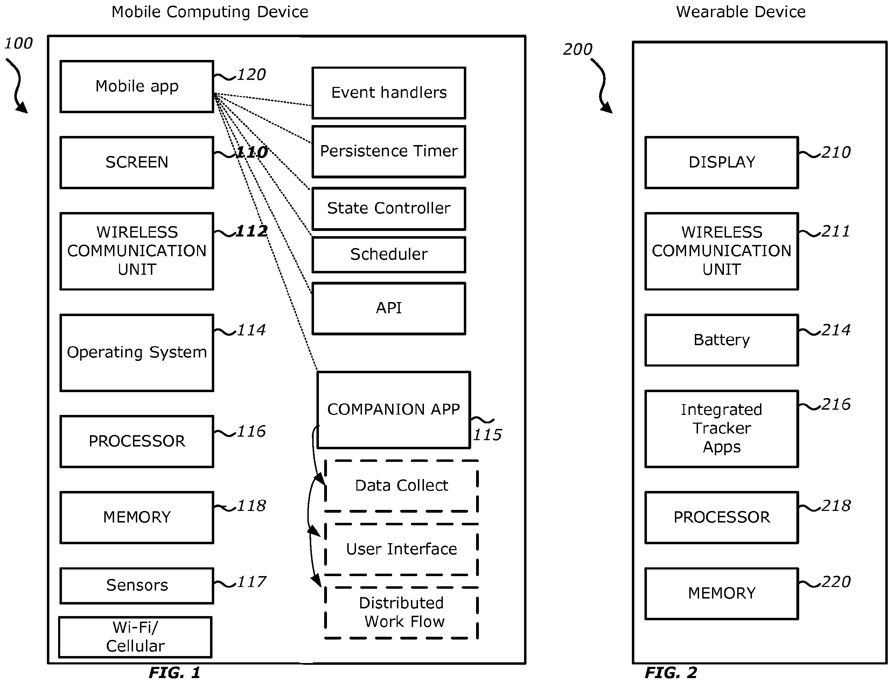

Figure 1 illustrates a diagram of an embodiment of a mobile app resident in a memory of a mobile computing device that generates and manages one or more companion apps. The mobile computing device 100 may have a mobile app 120 that generates one or more companion apps 115 as well as other mobile apps (that are not further discussed herein), a display screen 110, a wireless communication unit 112, an operating system 114, one or more processors 116, a memory 118, one or more sensors 117, a Wi-Fi and/or cellular circuit, a battery and many other components. Figure 2 illustrates a diagram of an embodiment of wearable device 200 having one or more integrated tracker apps 216 cooperating with the one or more companion apps 115. The wearable device 200 may have one or more integrated tracker apps 216, a display screen 210, a wireless communication unit 211, an operating system, one or more processors 218, a memory 220, one or more sensors, a battery and many other components.

Referring to figures 1 and 2, the mobile app 120, such as a Fitbit app, is resident in the memory 118 of the mobile computing device 100, such as a smart phone. The mobile app 120 has multiple components including one or more event handers, a persistence timer, a state controller, a scheduler, and an application programming interface. The mobile app 120 generates and manages one or more companion apps 115 in the memory 118 of the mobile computing device 100. The one or more companion apps 115 in the memory 118 of the mobile computing device 100 cooperate with one or more integrated tracker apps 216 in a memory 220 of the wearable device 200 in order to provide a combined functionality to the wearable device 200. In an embodiment, each companion app 115 is assigned with its own integrated tracker app 216 in the wearable device 200, such as a fitness tracker app.

The companion app 115 is in communication with the integrated tracker app 216 to provide functionality including i) perform data collection by the companion app 115 on behalf of the integrated tracker app 216, ii) facilitate settings updates for the integrated tracker app 216 on the wearable device 200 configured through a user interface of the companion app 115 displayed on a display screen 110 of the mobile computing device 100, and iii) extend capabilities of the integrated tracker app 216, such as by adding connectivity to the Internet to access Internet web services and/or enable browsing, and/or obtain and supply sensor data from one or more sensors 117 in the phone, such as GPS information, and/or perform a distributed workflow on behalf of an integrated tracker app 216, etc.

The one or more integrated tracker apps 216, such as a matching tracker app of: a golf app, a health app, a weather app, etc., are resident in the memory 220 of the wearable device 200 and are configured to wirelessly pair through a wireless communication circuit 211 of the wearable device 200 to the one or more companion apps 115 loaded in the memory 118 of the mobile computing device 100. The integrated tracker app 216 on the wearable device 200 submits requests for the one or more event handlers through the one or more application programming interfaces of the mobile app 120. The instructions for the integrated tracker app 216 are executed by one or more processors 218 located in the wearable device 200. The instructions for the companion app 115 are executed by one or more processors 116 located in the mobile computing device 100.

The companion app 115 is configured to run in the memory 118 of the mobile computing device 100 while the wearable device 200 is not running an executable file of the integrated tracker app 216 in order to allow the companion app 115 to perform, all of or just a subset of the following, functions including i) pre-fetching data on behalf of the integrated tracker app 216, ii) recording settings updates to the integrated tracker app 216 on the wearable device 200 that are configured through the user interface of the companion app 115 displayed on the display screen 110 of the mobile computing device 100, iii) extending a computational and memory storage capabilities available to the integrated tracker app 216 via a distributed workflow between the integrated tracker app 216 and the companion app 115, such as the companion app 115 making heavy computational loads with the processor 116 in the mobile computing device 100 and then sending the results to the integrated tracker app 216, etc., iv) extend capabilities of the integrated tracker app 216 by adding connectivity to the Internet to access On-line services and gather data on behalf of the integrated tracker app 216 via the integrated tracking app 216 submitting a request to a fetch application programming interface for the companion app 115, and v) obtain sensor data from one or more sensors 117 in the mobile computing device 100, such as GPS information, on behalf of the integrated tracker app 216, all while the wearable device 200 is not running the integrated tracker app 216. Thus, when the integrated tracker app 216 is not active and not consuming the battery of the wearable device 200, then the one or more companion apps 115 may be active and running to perform the above functions on behalf of the integrated tracker app 216.

Next, the state controller of the mobile app 120 regulates two main operational states of the companion app 115 i) loaded (e.g. running, listening for events from an event queue, and handling events), and ii) unloading (e.g. in the process of being killed). The scheduler cooperates with the state controller for the companion app 115 to coordinate activities between i) multiples instances of the companion app 115 loaded in the memory 118 of the mobile computing device 100 and ii) the one or more integrated tracker apps 216 running in the memory 220 of the wearable device 200. Note, typically if an app had cooperating portions on a watch and smart phone, then there was no need for an API for the two to communicate because internally the same company was scripting the code for the wearable side app and the phone side app. Here, third parties can code merely i) the companion app 115 or ii) the integrated tracker app 216 or iii) both under a license. A different company can code the companion app 115 than the integrated tracker app 216.

Each integrated tracker app 216 occupies less memory space, consumes less battery power, and consumes less computing cycles from the processor 218 on the wearable device 200 than if that integrated tracker app 216 performed all of the combined functionality given to the wearable device 200 from the integrated tracker app 216 cooperating with the companion app 115.

A third party integrated tracker app 216 can utilize the mobile computing device's 100 capabilities via the companion app 115 instances as opposed to interacting directly with the mobile computing device's operating system 114 or its corresponding mobile app 120 resident on the mobile computing device 100. The mobile app 120 generating the companion apps 115 is not part of an operating system 114 of the mobile computing device 100 but rather a standalone mobile app 120.

The companion app 115 can receive an indication message from the mobile app 120 resident on the mobile computing device 100 when the companion app 115 is using too many resources (CPU cycles, too many network requests, heap usage, etc.) and is significantly impacting on the mobile computing device's 100 (battery, data usage, responsiveness) performance. When the indication message comes from the operating system 114 to the mobile app 120 and then communicated over to the companion app 115, the companion app 115 then can deallocate some memory usage, such a cache space with stored information on behalf of the companion app 115, to use less resources on the mobile computing device 100. Thus, a user of a wearable device 200, will be able to enjoy the companion apps 115 with no significant impact on their smart phone's (battery, data usage, responsiveness) performance.

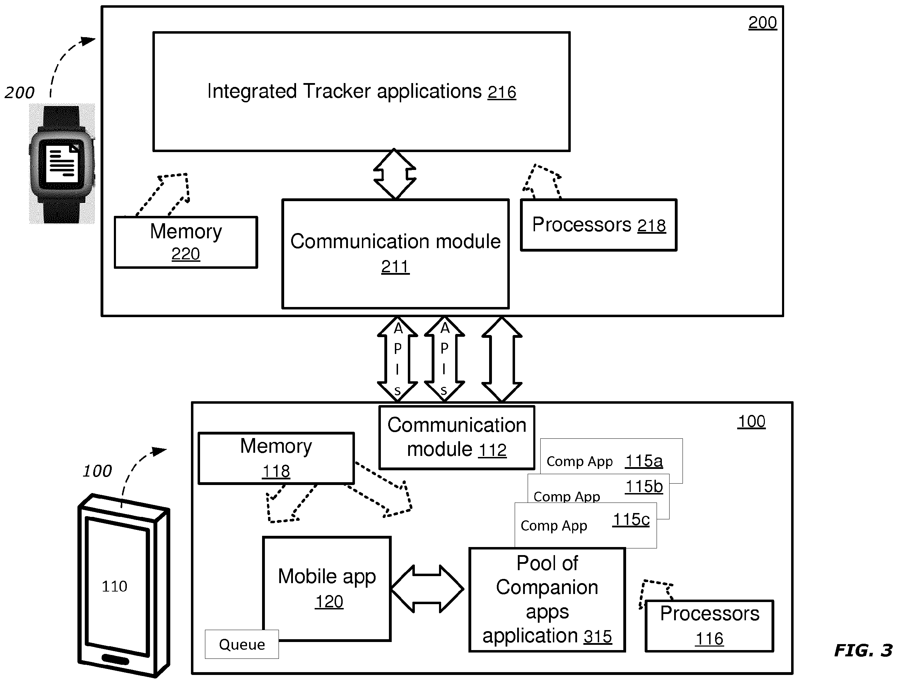

Figure 3 illustrates a diagram of an embodiment of the mobile app that is configured to run multiple instances of the companion app concurrently in the memory of the mobile computing device and form a pool of instances of companion apps that are running in the memory of the mobile computing device and cooperating via one or more application programming interfaces with multiple integrated tracker apps. The mobile computing device 100 may contain at least a memory 118, one or more processors 116, a communication module 112, and a mobile app 120 with one or more pools 315 of companion apps, such as a first companion app 115a, a second companion app 115b, and a third companion app 115c in a first pool 315. Each pool 315 may have multiple companion apps such as one to five companion apps. The wearable device 200 may contain at least a memory 220, one or more processors 218, a communication module 211, and one or more integrated tracker apps 216.

### Companion App Pool

The scheduler of the mobile app 120 may run multiple instances of the companion app concurrently in the memory 118 of the mobile computing device 100 and form a pool of instances of companion apps 315 that are running in the memory 118 of the mobile computing device 100. All of the multiple instances of the companion app in the pool 315, such as a first companion app 115a, a second companion app 115b, and a third companion app 115c, are related to each other in that one companion app may have priority/be promoted over another companion app. Also, each wearable device 200 in communication with the mobile device 100, via the communication module 211, will have its own pool of companion apps 315 that cooperate with the integrated tracker apps 216 on that wearable device 200. Thus, the companion apps in that pool 315 are associated with the one of more integrated tracker apps 216 from a same/assigned wearable device 200. For example, the first companion app 115a is associated with the first integrated tracker app, such as a weather app, on the first wearable device 200, and a second companion app 115b is associated with a second integrated tracker app, such as a golf app, on the first wearable device 200, and the third companion app 115c in the pool 315 is associated with a third integrated tracker app, such as a fitness app on the first wearable device 200.

In an embodiment, an integrated tracker app is always associated with one and only one companion app. The one to one ratio of companion apps to integrated tracker apps is easier for tracker app developers to work with; as opposed to, multiple companion apps working with one tracker app or multiple tracker apps from different third party's all working with a single companion app. This also prevents stampedes of generating too many companion apps in the case of multiple apps being registered for a "Significant Location Change" - a situation that becomes even worse when a user has multiple tracker devices all connected to the mobile computing device.

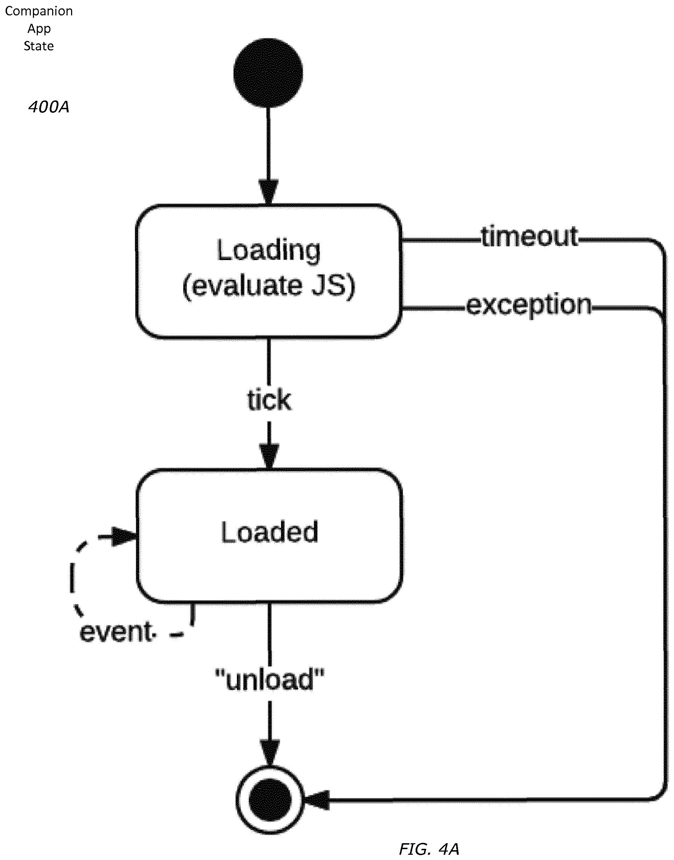

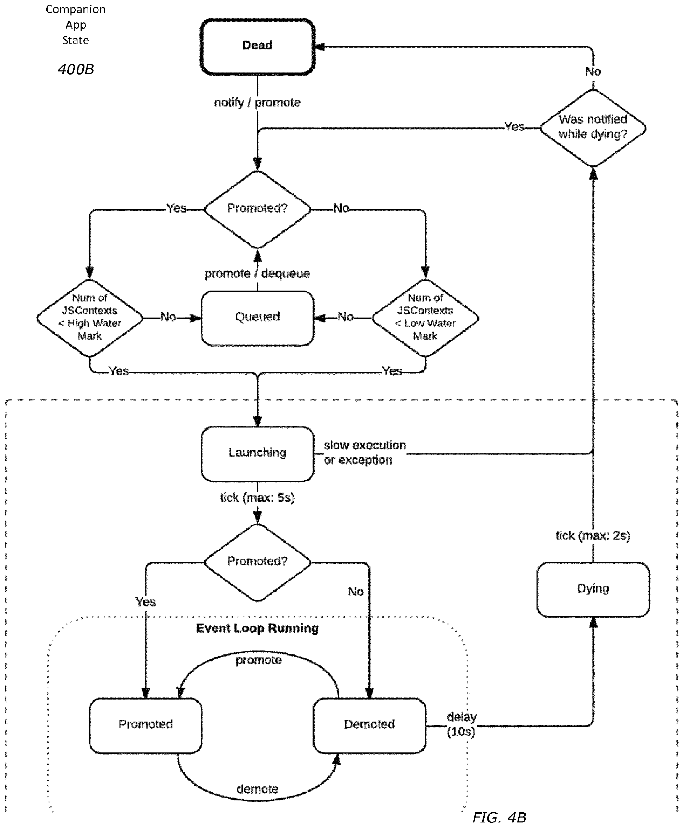

The scheduler of the mobile app 120 may limit an amount of instances of companion apps running concurrently/simultaneously in the pool 315 to a threshold set amount, such as five or less. The pool 315 contains all of the running companion apps for a specific wearable device in communication with the mobile app 120. When launching another instance of the companion app in the pool 315 would violate the threshold set amount of the pool, then the scheduler will put a next instance of the companion app into a priority queue. In the priority queue, promoted companion apps have priority over all other companion apps in a different sub state including a demoted companion app. (In general, see figure 4 for a discussion on states of operation of a companion app.) This priority queue can be persisted so that after a crash or user-termination of the mobile app (or suspension and termination under by the Operating System of the mobile computing device 100), this allows for a fair runtime distribution between multiple apps. Once a companion app leaves the pool 315, the scheduler will try to move the item on top of the priority queue into the pool 315.

### Preferential Schduling

The pool 315 can have two limits set for the threshold: a high water mark (HWM) and a low water mark (LWM). A conservative choice for the beginning would be (LWM=1, HWM=3), but a recommend choice to use is (LWM=2, HWM=5). Note, the threshold can be made adaptive to the device's hardware settings configuration.
- If COUNT(runningApps) >= LowWaterMark , no demoted companion apps will be added to the pool 315.
- If COUNT(runningApps) >= HighWaterMark , no companion apps at all will be added to the pool 315.

The scheduler of the companion app is also configured such that when an instance of a companion app is either i) launched in the memory 118 on the mobile computing device 100 and has been paired with/assigned to the integrated tracking app 216 or ii) queued to be launched and then intended to cooperate with the integrated tracking app 216, then all requests and communications associated with the integrated tracking app 216 are sent to that instance of the companion app rather than launching a second instance of the companion app in the pool 315 to handle a subsequent request from the integrated tracking app 216, which minimizes an amount of instances of companion apps launched concurrently in the pool 315. This reduces a higher memory usage, since more JavaScript coded companion app contexts have to be run if each instance merely handled a subset of requests from its matching integrated tracking app.

In an embodiment, running multiple companion apps concurrently in the companion pool 315 in the volatile memory 118 of the mobile computing device 100 is necessary in order to satisfy requirements of at least i) live and up to date data being available and used by the integrated tracker apps 216 when multiple integrated tracker apps 216 are running in the wearable device 200 and ii) pre-fetching of data and/or performing functions on behalf of multiple integrated tracker apps 216 without a lag negatively affecting a user experience. Note, using a high amount of memory increases the risk of being killed by the operating system and therefore risking the core mobile app experience. Therefore, the number of apps that can run simultaneously will be limited by the scheduler. This is a necessary but not sufficient condition for guaranteeing stability of the host mobile app 120; it does not prevent apps from destabilizing the host mobile app 120 in other ways (e.g. Application Not Responding (ANR), blowing up the heap, etc.). Code to stabilize the mobile app for Application Not Responding (ANR), blowing up the heap is also coded into the host mobile app 120.

Note, when two wearable devices connect to the mobile app, two companion pools are formed, and this replication will continue in a similar manner. In an embodiment, a single companion app can be associated with two or more integrated tracker apps, and vice versa.

Next, in an embodiment, each companion app is configured to provide integrated functionality on behalf of a corresponding integrated tracker app for any subset or combination of all three of i) perform data collection by the companion app on behalf of the integrated tracker app, ii) provide settings updates for the integrated tracker app on the wearable device configured through the user interface of the companion app displayed on the display screen of the mobile computing device, as well as iii) extend capabilities of the integrated tracker app.

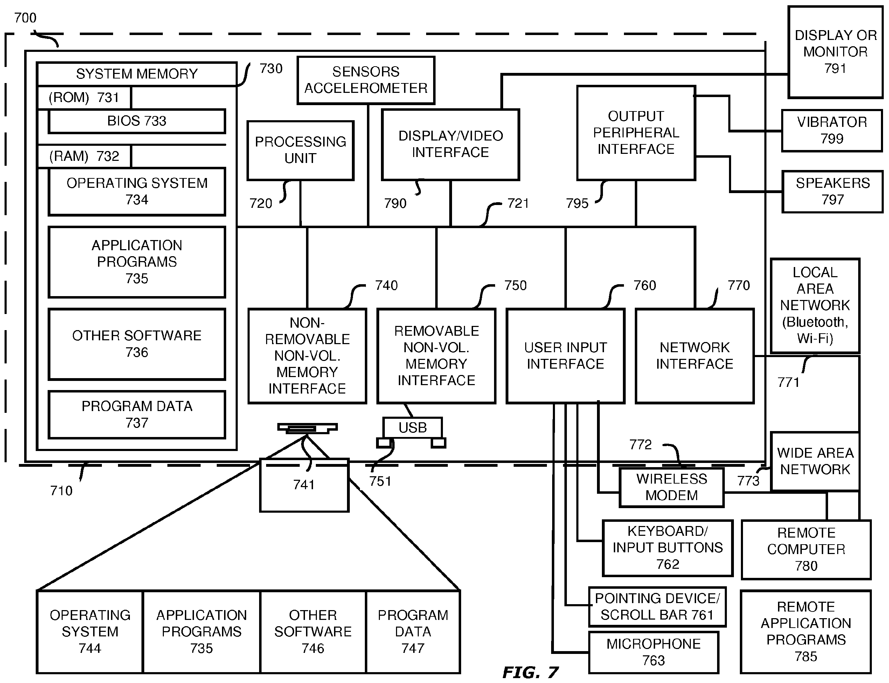

Each companion app can configure settings updates for its matching integrated tracker app on the wearable device i) through a user interface of the companion app displayed on a display screen of the mobile computing device and ii) through use of a diversity of user input mechanisms of the mobile computing device (See figure 7 for more example input mechanisms). The user input mechanisms may include any of a combination of a keyboard input, a voice recognition input, or a touch screen input for the mobile computing device, which allows a user of the matching integrated tracker app on the wearable device to take advantage of a bigger display screen and a diverse amount of user input mechanisms of the mobile computing device, via the user interface of the companion app, to make settings changes that are then conveyed and implemented on the matching integrated tracker app on the wearable device. As an example of settings changes, in an embodiment, the companion application may present a user interface on the display of the mobile computing device configured to allow the user to configure the pushbuttons of the wearable device to correspond to different functions within the integrated tracker app resident on the wearable device. Note, the user interface of companion app for settings configurations of the integrated tracker app is coded that when active on the display screen on the mobile device, the companion app captures and relays the settings changes in real-time, less than two seconds, with the wearable device (as soon as a setting is changed, there is a coded mechanism to propagate it to the watch and/or tracker device through the communication module).

### Continued functionality

Each integrated tracker app 216 is coded to run and operate even when the paired companion app may not be operational. This is similar to the coding for the companion app to collect data, perform calculations, and perform other functions on behalf of the integrated tracker app 216 even when the tracker app is not being executed. For example, the integrated tracker app 216 resident on the wearable device 200 is configured to collect the user's tracked data, such as health and fitness data and/or location data. The integrated tracker app 216 is coded that when disconnected with the companion app on the mobile computing device 100, then the integrated tracker app 216 is configured to continue to collect this information and continue to give the user of the wearable device 200 feedback on an activity that user is engaged in, via any of a display, a vibration, a turning on or off of lights, and an emitting of a sound. Thus, the integrated tracker app 216 may be disconnected with the companion app because the wearable device 200 and the mobile computing device 100 are outside the wireless communication range of the communication modules 211, 112 of each respective device and/or the mobile computing device 100 may be powered off but the integrated tracker app 216 is coded to continue to collect this information and continue to give the user of the wearable device 200 feedback on an activity that user is engaged in.

The tracker operating system of the wearable device 200 is coded to periodically check to see if the wireless communication circuit 112 of the mobile computing device 100 is within wireless range to establish a connection between the wireless communication circuit 211 of the wearable device 200 and the wireless communication circuit 112 of the mobile computing device 100. When the companion app on the mobile computing device 100 is loaded in the memory 118 and within range to establish the connection, the integrated tracker app 216 can migrate all of the tracked data from its memory 220 when the two applications communicate with each other, and then the companion app is configured to synchronize the tracked data from the wearable device 200 into the mobile computing device 100. Note, the independence of the integrated tracker app 216 being able to run and be functional while the mobile app is not running, and vice versa allows various benefits. For example, this allows a use case where firmware can be built generally across platforms for third party apps, and different instances of the mobile app need not be built specifically for each third party app. Note, again, the combined functionality of the companion app and integrated tracking app 216 provides additional functionality beyond what the integrated tracking app 216 can perform merely by itself and with the hardware and software resources available on the wearable device 200. However, the core functions of the integrated tracker app 216 are coded to be able to run and collect data when not connected to the companion app. For example, one combined function of the two apps is further extending a computational and memory storage capabilities available to the integrated tracker app 216 by the companion app performing a distributed workflow using the second processor 116 and memory 118 of the mobile computing device 100 on behalf of the integrated tracker app 216. When disconnected the integrated tracking app 216 may be coded to perform all of the same calculations; however, the response time may lag compared to when the two apps, the companion app and the integrated tracker app 216 cooperate to provide the combined functionality. Another combined function is the companion app being coded to display a user interface to configure settings using the bigger display screen 110 and diverse input mechanisms of the mobile computing device 100. The integrated tracker app 216 is also coded to make all of the settings changes by merely using the wearable device's 200 resources but it is much more convenient using the mobile computing device's 100 resources.

Briefly referring to figure 4, a companion app's life cycle may include main operational states of i) loaded into the volatile memory, and ii) unloading out of the volatile memory of the mobile computing device 100. The sub states may include at least i) Dead, ii) Dying, iii) Queued, iv) Launched, v) Promoted, and vi) Demoted.

### Application Programming Interfaces (APIs)

Next, referring to figure 3, the one or more APIs 317 facilitate communicating, submitting requests, and passing data between the companion app and the integrated tracker app 216 over the wireless communication channel established by the communication modules 211, 112. Additional communication channels 319 may exist between the wearable device 200 and the mobile computing device 100 as well. Note, the one or more APIs 317 are i) not part of or ii) for the operating system but rather are APIs for the mobile app 120 for the companion apps. Each API in the set of APIs 317 may use a nomenclature convention (e.g. "ActionListener" and "ActionEvent") to relate and organize event concerns. An API 317 also defines methods of communication between the components within the companion app and the one or more integrated tracker apps 216 attempting to cooperate with instances of the companion app. Developers may create and publish their own APIs 317 for communications between the companion app and the integrated tracker app 216.

### Properties and Methods

As some examples, the following properties can be exposed on the global mobile app object of the mobile app 120 to the developer of an integrated tracker app 216. Some examples are:
1. Launch reasons for a companion app(): LaunchReasons API
   a. trackerAppLaunchedByUser: any
   b. IocationChanged: { position: Position }
   c. a launch reason set specifically by the integrated tracker app 216
2. .yield(): void API
   a. If the companion app is running in the "demoted" state, calling this API will move the companion app to the "dying" state. This allows companion apps to vacate their slot quicker once they are done prefetching their data (e.g. after getting weather from a server and adding it to the FileTransferQueue) and is considered 'good behavior'.
   b. If the companion app is running in any other state of operation, calling this API will not have an effect.
   Note, the companion app is coded to compensate for when developers of integrated tracker apps 216 set up a Promise chain for their background tasks that ends in "yield". While this is executing, the user can have launched the integrated tracker app 216, and terminating any interactive communication could negatively impact the user experience.
3. getCurrentPosition() method. - Geolocation API
   a. The integrated tracker app 216 through this API to companion app can obtain sensor data on the mobile computing device 100. For example, the integrated tracker app 216 through this API to companion app can obtain the GPS sensor data.
4. getComponent() method
   a. The integrated tracker app 216 through this API to companion app can utilize components on the mobile computing device 100 on behalf of the wearable device 200. For example, the integrated tracker app 216 through this API to companion app can access to the camera and utilize the camera function as part of the integrated tracker app 216 and/or for example utilize the camera roll from the settings system so that the user can provide an image.
5. getwebservices() method
   a. The integrated tracker app 216 through this API to companion app can access web services that require the mobile app to authenticate without asking the user of the wearable device 200 to reauthenticate. The integrated tracker app 216 through this API to companion app also can create Websocket connections to HTTP(S) servers.
6. getexchangemessages () method
   a. The integrated tracker app 216 through this API to companion app can exchange messages and requests with the mobile app via the Interactive Communication system (JS API).
7. localstorage () method
   a. The integrated tracker app 216 through this API to companion app can use the localstorage API which stays persisted even across app updates to store information on the memory of the mobile computing device 100 on behalf of the integrated tracker app 216 on the wearable device 200.

These are merely some examples of the APIs set up between the integrated tracker app 216 and the companion app.

Figure 4A illustrates a state diagram of an embodiment of a companion app's life cycle that include main operational states of i) loaded into the volatile memory, and ii) unloading out of the volatile memory of the mobile computing device. Figure 4B illustrates a state diagram of an embodiment of a companion app's life cycle that include sub states of at least i) Dead, ii) Dying, iii) Queued, iv) Launched, v) Promoted, and vi) Demoted.

Referring to the operational states 400B in figure 4B, the state controller may transition the companion app into and out of sub states of the main operational state of loaded in the memory. The state controller may transition the companion app from Dead to Promoted, Queued, or Launched. The sub states may include the following.
i) Queued - in general, the state controller has triggered the companion app to be executed but it is awaiting an available slot by the scheduler. See the discussion about the companion pool.
ii) Launched - in general, the companion app is being executed and run in the memory of the mobile computing device.
iii) Promoted - in general, the companion app is running concurrently with other companion apps and has priority for scheduling and servicing over one or more of the companion apps, and in addition the companion app will keep this sub state context running until an event triggers its demotion.
iv) Demoted - in general, the companion app is handling events but no corresponding integrated tracker app is currently running in the memory of wearable device.

The state controller may also transition the companion app into and out of sub states of the main operational state of unloading in the memory. The sub states can include the following.
i) Dead - in general, the state controller is awaiting a launch event or notice to generate an instance of the companion app.
ii) Dying - in general, the state controller is coded to expect a communication from the integrated tracker app that the integrated tracker app will not send any more events during this life cycle of the companion app; and accordingly, the companion app should be unloaded from the memory of the mobile computing device when all of the event handlers for the companion app return their results back to the integrated tracker app.

### More detail on the operational states of a companion app

- Dead: the companion app is present in the mobile app's registry, but first a notification has occur which will then launch a context of the companion app into the memory. Operating in the background, the operating system may be more likely to terminate the companion apps. The companion app on the mobile device may receive a timely event notice from the mobile app on the mobile computing device before the state controller for the companion app transitions the companion app to a dead operational state in order to improve a functioning of the mobile computing device in at least two ways i) so that the companion app is configured to save state in response to receiving the timely event notice; and thus, does not have to continuously save state all the time, as well as ii) the companion app is configured to make network requests merely when the companion app is likely to have enough time to complete this task prior to be transitioned to the dead operational state. Thus, a developer's companion app will receive a timely event before they are terminated so that they can save state (and does not have to save state all the time), and make network requests merely when the network requests are likely to have enough time to be completed. The timely event notice allows the companion app to minimize 1) a need for CPU cycles from the second processer and 2) a cumulative amount of network requests made by the companion app on the mobile computing device. Due to the OS being more apt to kill an app in the background, companion apps are coded to handle the case when they are "launched in the background" to pre-fetch data or "launched by user" to extend the capabilities of the integrated tracker app, such as by adding connectivity to the internet.
- Queued: something, such as a notice or event, triggered the companion app to be executed. However executing the actual generation of the companion app at this time would have violated the pool limits, so this instance of the companion app has to wait for a slot to become available. (See more in the companion pool discussion).
- Launching: The JavaScript context of the companion app is being created and the bundled JavaScript file is being executed. Note, on an exception (or the JavaScript taking too long to being parsed and executed), then the companion app can be terminated immediately without going through the dying state.

The state controller for the companion app may trigger an operational state of launched in the memory on the mobile computing device for various detected situations including the following.
i) In response a user launching the integrated tracker app on the wearable device, then a first message is communicated to the state controller to launch the companion app.
ii) When the companion app has requested to be woken up at periodic time intervals because the tracker app and companion have not been in communication for a threshold amount of time, then the companion app is launched in order to pre-fetch data that will be used by the first tracker app once it is launched on the wearable device. Note, the persistence timer tracking the threshold amount of time should merely fire when the wearable device is connected to the mobile computing device. When the wearable device disconnects, the system can stop delivering those events. When the wearable device reconnects, the state controller will wake-up the companion app as soon as possible if the threshold amount of time tracked by the persistence timer has expired. The threshold amount of time may be a configurable setting.
iii) When the monitoring routine of the companion app communicates the second message that a significant location change has happened for the wearable device. Note, the significant location change is equal to or above a location change threshold set by the companion app. The significant geographic location change for the wearable device may include a detection of i) a change in cell towers, servicing phone calls, text messages, and/or Internet usage of the wearable device, ii) a change in GPS coordinates, iii) a change in tracked physical data of the wearer, from for example, the integrated tracker app itself, indicating that the user has traveled equal to or greater than the location change threshold set by the companion app, and iv) etc. A significant location change may be the user moving at least 2 miles / 3 kilometers. However, multiple levels of "significant location" may be set in the settings such as 2.0 and 1.0 miles. Note, to limit rapid generation of events when the user is moving fast, the settings can be set to not update the app more than once every 15 minutes so if the user is moving fast, the updates will be limited to one every 15 minutes.
   The precision provided in these updates can be low (cellular tower / wifi cell). If the developer of the integrated tracker app wants a higher accuracy, the integrated tracker app can be coded to use, for example, the location API.
   Significant location changes should merely be delivered when the wearable device is connected to the mobile computing device. When the wearable device disconnects, the system can stop delivering those events. When the user significantly changes their location, a "location change" event might be dispatched from a fitbit.significantLocationUpdates object.
iv) When the integrated tracker app receives a notification communication from the Internet/cloud. The state controller wakes up and launches the companion app so that the notification can be acted upon by the first tracker app without any delay. The state controller can launch a companion apps to process notifications that are sent by the developer of the integrated tracker app to the Fitbit cloud platform.

Whenever a Notification launch event is received the Notification launch event may either be delivered as a JavaScript event into the companion app (if already running) or be stored and passed as a Launch Reason to the companion app once the companion app can be launched (see the Companion Pool for further details). Some of these events might come with a payload (e.g. a GPS coordinate) or cause the companion app to be "promoted."

The companion app may trigger an operational state of launched on the mobile computing device when the tracker app has been launched on the wearable device by the user so that the companion app can pre-fetch data from an Internet source and/or sensor data from sensors on the mobile computing device and minimize a time period it takes for the tracker app to display up to date/current information on a display of the wearable device when the user launches the tracker app on the wearable device.

Note, there may be no event for the "launch" transition. When the script is evaluated, it can assume to have been launched f the companion app throws an exception (or causes a SyntaxError) while the system evaluates the JavaScript file (or when it takes longer than 5 seconds to evaluate), then the companion app will be unloaded without going through the unload transition.
- Promoted: A JavaScript context of the companion app that has priority. The mobile app will attempt to keep this context of the companion app running until an event triggers the companion app's demotion.
- Demoted: A JavaScript context of the companion app that is handling events but no corresponding integrated tracker app is running. The mobile app will transition this context into the Dying state after a certain time (e.g. 10 seconds). Optionally, the mobile app might close the companion app because the mobile app detects that no timers are scheduled, the event-queue is empty, and no I/O handles are left open.

### Revisiting Promotion and Demotion

A promoted companion app should be transitioned to launched as soon as possible and run for as long as possible (until it is transitioned to "demoted"). In general, "promoted" and " demoted" events will be coupled to integrated tracker app lifecycle events that are received from the tracker.
1. When the matching tracker app is active and running the "promote" transition will be triggered by the state controller.
2. When the matching tracker app is closed on the wearable device the "demote" transition will be triggered.

Other conditions can affect whether a promoted companion app is transitioned to demoted or not. For example, if background apps are running on the tracker device, and/or if app settings require the companion app to run (or have the companion app as the logic driver), then the companion app will not be demoted until these other conditions have been resolved.

The state controller can also determine companion app operational state based on a behavior of its matching integrated tracker app. For example, integrated tracker apps that use the yield-API could have higher priority in the queue. Likewise, an integrated tracker app that seems to be running every time the host mobile app crashes can have a lower priority in the queue.
- Dying: Upon entering this state, the mobile app will send an "unload" event to the context of the companion app to inform the companion app that it will be unloaded. After this the mobile app will not send any events to this JavaScript context of the companion app anymore. Once all event handlers return (synchronously), then the companion app will be unloaded from memory. Also, if a wearable device (or tracker app) hasn't been connected for a while (e.g. 24 hours), then the state controller may decide to not run the companion app and dissolve that companion app pool. The companion app can also be terminated if the event handlers registered for the "unload" event take longer than 2 seconds in total. "Unload" can be called when the system stops executing the JavaScript runtime and removes the companion app from memory. There will not be another tick after the event handler got called - the behavior of asynchronous APIs is undefined.

Overall, the companion app can share a single execution context for all states. The companion app could also have isolated execution contexts per state. Note, multiple sub states have an advantage in that if the companion app could only be in a single state, transition between states are costly as JavaScript has to be parsed and evaluated every time.

Referring to the operational states 400A in figure 4A, the JavaScript companion apps are designed with a simple lifecycle of two main operational states: loaded and unloading to provide a predictable lifecycle for the companion apps. The operational states are made robust with many sub states within the main operational states. This allows a developer of the integrated tracker app to write simple event handlers that are easy to write, debug and maintain and are predictable in behavior. Also the predictable lifecycle for companion apps is generally the same for a companion app independent of what operating system the mobile computing device is using such as Android OS or iOS. Transitions between the loading/loaded states can be exposed as JavaScript events on the global "mobile app" object. In general, a developer of the integrated tracker app can write code for one companion app that can operate on all types wearable devices from a specific manufacturer and that runs apps with minimal fragmentation.

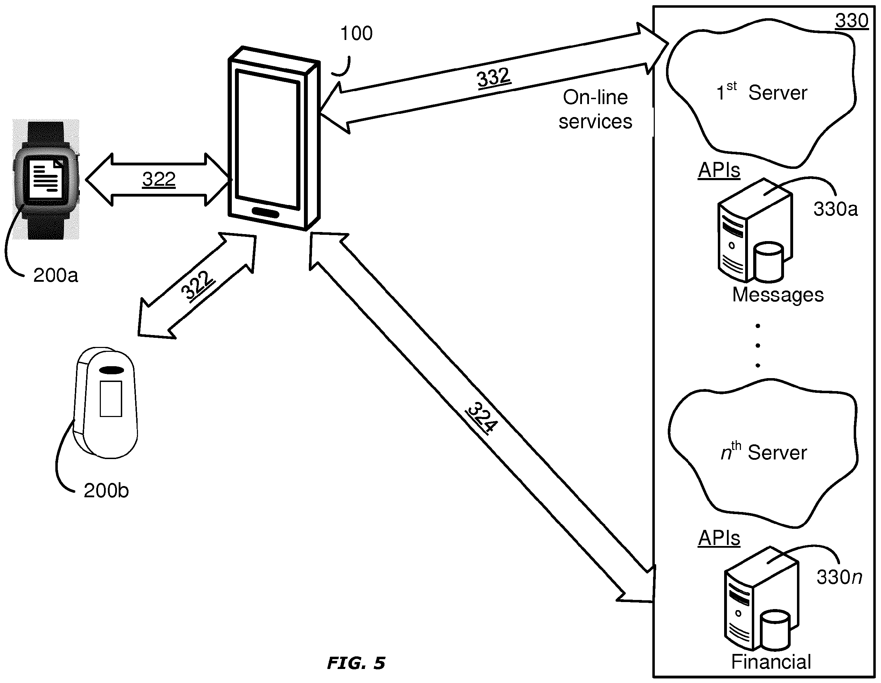

Figure 5 illustrates a diagram of an embodiment of a companion app extending capabilities of the integrated tracker app by interacting with the Internet web services on behalf of the integrated tracker app even when the wearable device is not running the integrated tracker app.

The wearable electronic devices 200a & 200b can be communicatively coupled through a short-ranged wireless connection 322, such a Bluetooth connection, with the mobile computing device 100. Thus, the wearable electronic devices 200a & 200b and the mobile computing device 100 can send and receive signals from each other such as requests and responses through the wireless connections 322. Additionally or alternatively, the wearable electronic devices 200a & 200b can be communicatively coupled through a wireless connection 332, 324 directly to a partner server 330a - 330n and bypass the mobile computing device 100. However in most cases, the wearable electronic devices 200a & 200b will communicate through the mobile computing device 100.

A first companion app can run in the memory of the mobile computing device 100 while the first wearable device 200a is not running an executable file of the first integrated tracker app. The first companion app is running in order to allow the first companion app to extend capabilities of the first integrated tracker app by interacting with the Internet web services, such as a partner server 330a - 330n, on behalf of the first integrated tracker app while the first wearable device 200a is not running the first integrated tracker app. For example, the first companion app is configured to access Internet web services (e.g. Weather, Sports, Stocks, etc.) and gather data on behalf of the first integrated tracker app via the first integrated tracking app submitting a request via a fetch API for the first companion app. Concurrently, a second companion app can run in the memory of the mobile computing device 100 and cooperate with an active integrated tracker app in memory of the first wearable device 200a. Concurrently, a third companion app can run in the memory of the mobile computing device 100 and cooperate with an active integrated tracker app in memory of the second wearable device 200b.

Likewise, the companion app is configured to cooperate with and leverage an authorization proxy for an existing web service (e.g. Gmail, Yahoo, Uber, Nest) to obtain an authentication token to access the user of the wearable device's protected resources at a third party service (e.g. Nest, Uber, Facebook, etc.) without asking the user of the wearable device to type their login or password on the wearable device. For example, the third companion app can cooperate with and leverage an authorization proxy for an existing web service on behalf of an integrated tracker app in memory of the second wearable device 200b without making its user reauthenticate but by merely using the token. The third companion app may then use the protected resources from the third party service to either i) deliver content back to the integrated tracker app and/or ii) conduct a financial transaction for the user of the integrated tracker app.

In an embodiment, the companion app may use an OAuth. The OAuth (Open Authorization) can be an open standard for token-based authentication and authorization on the Internet. The OAuth used allows an end user's account information to be used by third-party services, such as Facebook hosted on partner server 330a, without exposing the user's password. The OAuth essentially allows access tokens to be issued to third-party clients by an authorization server, with the approval of the resource owner. The third party then uses the access token to access the protected resources hosted by the resource server. Thus, the companion app can be coded to access web services that require local host app authentication without asking the user to reauthenticate.

The host mobile app for the companion app can communicate with multiple trackers. The first integrated tracker app on the first tracker device 200a may be a game app and engage in game play with a second integrated tracker app on a second tracker device 200b. The companion apps running in the background of the mobile computing device 100 can perform a distributed workflow with each integrated tracker app. Thus, the mobile app may talk to multiple tracker devices at the same time. The host mobile app can support this use-case by providing APIs that allow IPC (like the "postMessage" API that is used to communicate between windows in web browsers).

### Networked environment

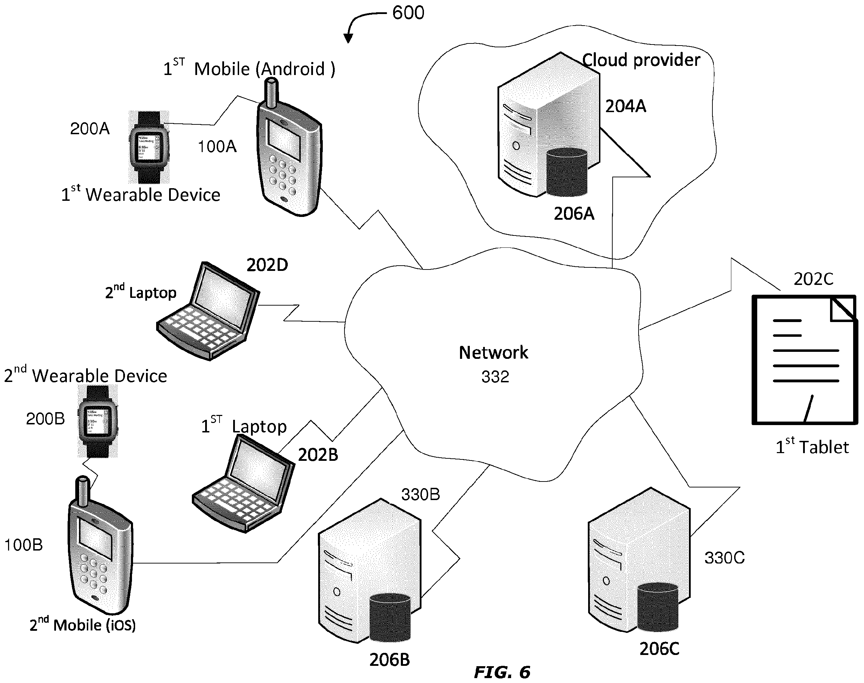

Figure 6 illustrates two or more wearable electronic devices communicating with other electronic devices on a network in accordance with some embodiments. The wearable electronic devices 200A, 200B may remotely access and/or communicate with other devices on a network in accordance with some embodiments. The network environment 600 has a communications network 322 that connects server computing systems 204A, 330B, and 330C, and at least one or more client computing systems 100A, 100B, 202B to 202D, as well as 200A and 200B. As shown, there may be many server computing systems 204A, 330B, and 330C and many client computing systems 100A, 100B, 202B to 202D, as well as 200A and 200B connected to each other via the network 322, which may be, for example, the Internet. The cloud-based server 204A can be coupled to two or more wearable electronic devices 200A and 200B and can bidirectionally communicate with the two or more mobile electronic devices 100A, 100B. The cloud-based server 204A can cooperate and supply additional information on each user to the mobile app.

Note, the network 322 might be or also include one or more of: an optical network, a cellular network, the Internet, a Local Area Network (LAN), Wide Area Network (WAN), satellite link, fiber network, cable network, or a combination of these and/or others. It is to be further appreciated that the use of the terms client computing system and server computing system is for clarity in specifying who generally initiates a communication (the client computing system) and who responds (the server computing system). No hierarchy is implied unless explicitly stated. Both functions may be in a single communicating device, in which case the client-server and server-client relationship may be viewed as peer-to-peer. Thus, if two systems such as the client computing system 100A and the server computing system 204A can both initiate and respond to communications, their communication may be viewed as peer-to-peer. Likewise, communications between the server computing systems 204A, 330B, and 330C, and the client computing systems 202A and 202C may be viewed as peer-to-peer if each such communicating device is capable of initiation and response to communication. Additionally, server computing systems 204A, 330B, and 330C, also have circuitry and software to communication with each other across the network 322. One or more of the server computing systems 204A, 330B, and 330C, may be associated with a database such as, for example, the databases 206A, 206B, and 206C. Each server may have one or more instances of a virtual server running on that physical server and multiple virtual instances may be implemented by the design. A firewall may be established between a client computing system 100A and the network 322 to protect data integrity on the client computing systems 100A, 200A. Each server computing system 204A, 330B, and 330C may have one or more firewalls.

A cloud provider service, such as computer server 204A and database 206A, can install and operate application software in the cloud and users can access the software service from the client devices. Cloud users who have a site in the cloud may not solely manage the cloud infrastructure and platform where the application runs. Thus, the multiple servers and databases in the cloud platform may be shared hardware where the user is given a certain amount of dedicate use of these resources. The user's cloud-based site is given a virtual amount of dedicated space and bandwidth in the cloud. Cloud applications can be different from other applications in their scalability, which can be achieved by cloning tasks onto multiple virtual machines at run-time to meet changing work demand. Load balancers distribute the work over the set of virtual machines. This process is transparent to the cloud user, who sees only a single access point.

The cloud-based remote access is coded to utilize a protocol, such as Hypertext Transfer Protocol (HTTP), to engage in a request and response cycle with both a mobile device application resident on a client device as well as a web-browser application resident on the client device. The cloud-based remote access for a wearable electronic device can be accessed by a mobile device, a desktop, a tablet device, and other similar devices, anytime, anywhere. Thus, the cloud-based remote access to a wearable electronic device hosted on a cloud-based provider site is coded to engage in 1) the request and response cycle from all web browser based applications, 2) SMS/twitter based request and response message exchanges, 3) the request and response cycle from a dedicated on-line server, 4) the request and response cycle directly between a native mobile application resident on a client device and the cloud-based remote access to a wearable electronic device, and 5) combinations of these.

In an embodiment, the server computing system 204A may also include a server engine, a web page management component, a content management component, and a database management component. The server engine performs basic processing and operating system level tasks. The web page management component handles creation and display or routing of web pages or screens associated with receiving and providing digital content and digital advertisements. Users may access the server-computing device by means of a URL associated therewith. The content management component handles most of the functions in the embodiments described herein. The database management component includes storage and retrieval tasks with respect to the database, queries to the database, and storage of data.

An embodiment of a server computing system to display information, such as a web page, etc. is discussed. An application including any program modules, apps, services, processes, and other similar software executable when executed on the server computing system 204A, causes the server computing system 204A to display windows and user interface screens on a portion of a media space, such as a web page. A user via a browser from the client computing system 100A may interact with the web page or the app hosted on the server computing system 204A, and then supply input to the query/fields and/or service presented by a user interface of the application. The web page may be served by a web server computing system 204A on any Hypertext Markup Language (HTML) or Wireless Access Protocol (WAP) enabled client computing system 100A or any equivalent thereof. For example, the client mobile computing system 100A may be a wearable electronic device, smart phone, a touch pad, a laptop, a netbook, etc. The client computing system 100A may host a browser, a mobile application, and/or watch specific application to interact with the server computing system 204A. Each application has a code scripted to perform the functions that the software component is coded to carry out such as presenting fields and icons to take details of desired information. Algorithms, routines, and engines within the server computing system 204A take the information from the presenting fields and icons and put that information into an appropriate storage medium such as a database. A comparison wizard is scripted to refer to a database and make use of such data. The applications may be hosted on the server computing system 204A and served to the browser of the client computing system 100A. The applications then serve pages that allow entry of details and further pages that allow entry of more details.

### Computing System

Figure 7 illustrates a computing system that can be part of one or more of the wearable electronic devices, one or more mobile computing devices, and server systems, in accordance with some embodiments. With reference to Figure 7, components of the computing system 810 may include, but are not limited to, a processing unit 820 having one or more processing cores, a system memory 830, and a system bus 821 that couples various system components including the system memory to the processing unit 820. The system bus 821 may be any of several types of bus structures including a memory bus or memory controller, a peripheral bus, and a local bus using any of a variety of bus architectures.

Computing system 810 typically includes a variety of computing machine-readable media. Computing machine-readable media can be any available media that can be accessed by computing system 810 and includes both volatile and nonvolatile media, removable and non-removable media. By way of example, and not limitation, computing machine-readable mediums uses include storage of information, such as computer readable instructions, data structures, other executable software or other data. Computer storage mediums include, but are not limited to, RAM, ROM, EEPROM, flash memory or other memory technology, CD-ROM, digital versatile disks (DVD) or other optical disk storage, magnetic cassettes, magnetic tape, magnetic disk storage or other magnetic storage devices, or any other tangible medium which can be used to store the desired information and which can be accessed by computing device 800. Transitory media such as wireless channels are not included in the storage media. Communication media typically embodies computer readable instructions, data structures, other executable software, or other transport mechanism and includes any information delivery media.

The system memory 830 includes computer storage media in the form of volatile and/or nonvolatile memory such as read only memory (ROM) 831 and random access memory (RAM) 832. A basic input/output system 833 (BIOS), containing the basic routines that help to transfer information between elements within computing system 810, such as during start-up, is typically stored in ROM 831. RAM 832 typically contains data and/or software that are immediately accessible to and/or presently being operated on by processing unit 820. By way of example, and not limitation, Figure 7 illustrates that RAM can include a portion of the operating system 834, other executable software 836, and program data 837.

The computing system 810 may also include other removable/non-removable volatile/nonvolatile computer storage media. By way of example only, Figure 7 illustrates a solid-state memory 841. Other removable/non-removable, volatile/nonvolatile computer storage media that can be used in the exemplary operating environment include, but are not limited to, USB drives and devices, flash memory cards, solid state RAM, solid state ROM, and the like. The solid-state memory 841 is typically connected to the system bus 821 through a non-removable memory interface such as interface 840, and USB drive 851 is typically connected to the system bus 821 by a removable memory interface, such as interface 850.

As an example, the computer readable storage medium 841 stores Operating System software for smart watches to cooperate with both Android OS and iOS.

The drives and their associated computer storage media discussed above and illustrated in Figure 7, provide storage of computer readable instructions, data structures, other executable software and other data for the computing system 810. In Figure 7, for example, the solid state memory 841 is illustrated for storing operating system 844, other executable software 846, and program data 847. Note that these components can either be the same as or different from operating system 834, other executable software 836, and program data 837. Operating system 844, other executable software 846, and program data 847 are given different numbers here to illustrate that, at a minimum, they are different copies. In an example, the operating system can be a customized Free RTOS kernel that can communicate with Android and iOS applications using Bluetooth, Wi-Fi, cellular or other communication methodology.

A user may enter commands and information into the computing system 810 through input devices such as a keyboard, touchscreen, or even push button input component 862, a microphone 863, a pointing device and/or scrolling input component 861, such as a mouse, trackball or touch pad. The microphone 863 may cooperate with speech recognition software. These and other input devices are often connected to the processing unit 820 through a user input interface 860 that is coupled to the system bus, but may be connected by other interface and bus structures, such as a parallel port, game port or a universal serial bus (USB). A display monitor 891 or other type of display screen device is also connected to the system bus 821 via an interface, such as a display and video interface 890. In addition to the monitor, computing devices may also include other peripheral output devices such as speakers 897, a vibrator 899, and other output device, which may be connected through an output peripheral interface 890.

The computing system 810 may operate in a networked environment using logical connections to one or more remote computers/client devices, such as a remote computing device 880. The remote computing device 880 may be a wearable electronic device, a personal computer, a hand-held device, a server, a router, a network PC, a peer device or other common network node, and typically includes many or all of the elements described above relative to the computing system 810. The logical connections depicted in Figure 7 include a local area network (LAN) 871 and a wide area network (WAN) 873, but may also include other networks. Such networking environments are commonplace in offices, enterprise-wide computer networks, intranets and the Internet. A browser application may be resident on the computing device and stored in the memory.

When used in a LAN networking environment, the computing system 810 is connected to the LAN 871 through a network interface or adapter 870, which can be a Bluetooth or Wi-Fi adapter. When used in a WAN networking environment, the computing system 810 typically includes a modem 872, e.g., a wireless network, or other means for establishing communications over the WAN 873, such as the Internet. The wireless modem 872, which may be internal or external, may be connected to the system bus 821 via the user-input interface 860, or other appropriate mechanism. In a networked environment, other software depicted relative to the computing system 810, or portions thereof, may be stored in the remote memory storage device. By way of example, and not limitation, Figure 7 illustrates remote application programs 885 as residing on remote computing device 880. It will be appreciated that the network connections shown are exemplary and other means of establishing a communications link between the computing devices may be used.

As discussed, the computing system may include a processor, a memory, a built in battery to power the computing device, an AC power input to charge the battery, a display screen, a built-in Wi-Fi circuitry to wirelessly communicate with a remote computing device connected to network.

It should be noted that the present design can be carried out on a general purpose computing system such as that described with respect to Figure 7. However, the present design can be carried out on a distributed system in which different portions of the present design are carried out on different parts of the distributed computing system.

Another device that may be coupled to bus 811 is a power supply such as a battery and Alternating Current adapter circuit. As discussed above, the DC power supply may be a battery, a fuel cell, or similar DC power source that needs to be recharged on a periodic basis. The wireless communication module 872 may employ a Wireless Application Protocol to establish a wireless communication channel. The wireless communication module 872 may implement a wireless networking standard.

Examples of mobile computing devices may be a tablet, a laptop computer, a smart phone, a personal digital assistant, or other similar device with on board processing power and wireless communications ability that is powered by a Direct Current (DC) power source that supplies DC voltage to the mobile device and that is solely within the mobile computing device and needs to be recharged on a periodic basis, such as a fuel cell or a battery.

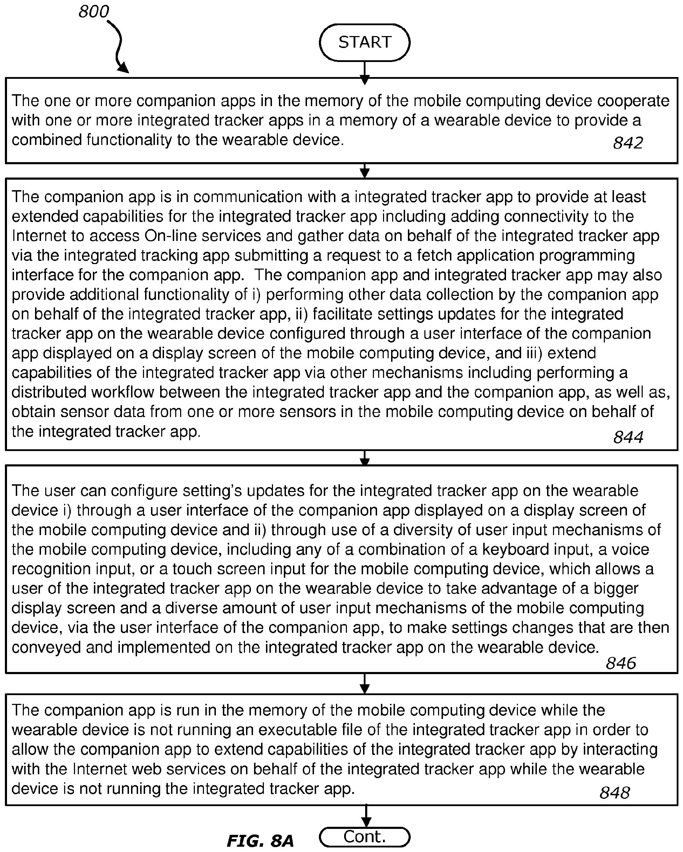

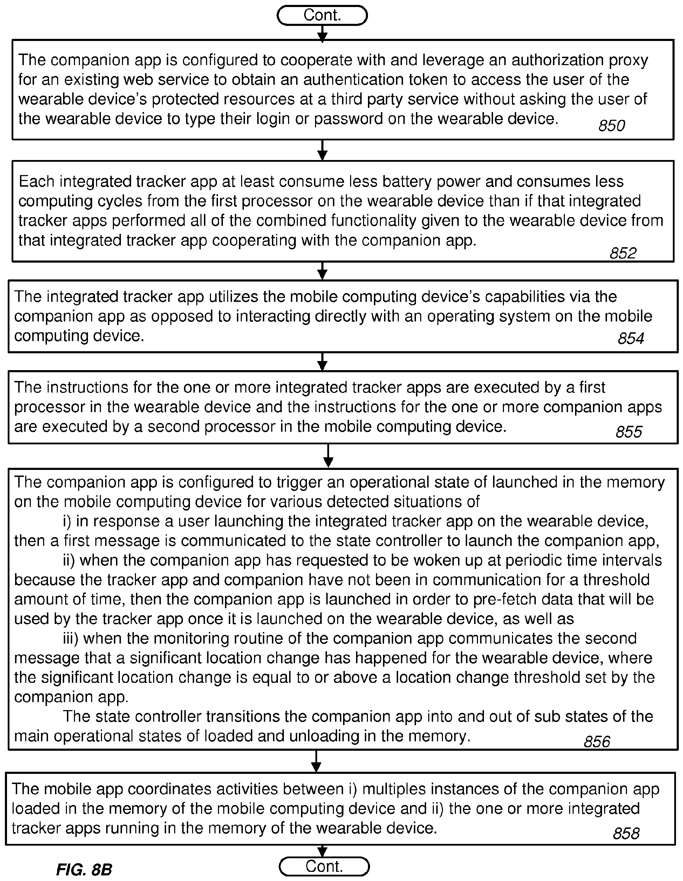

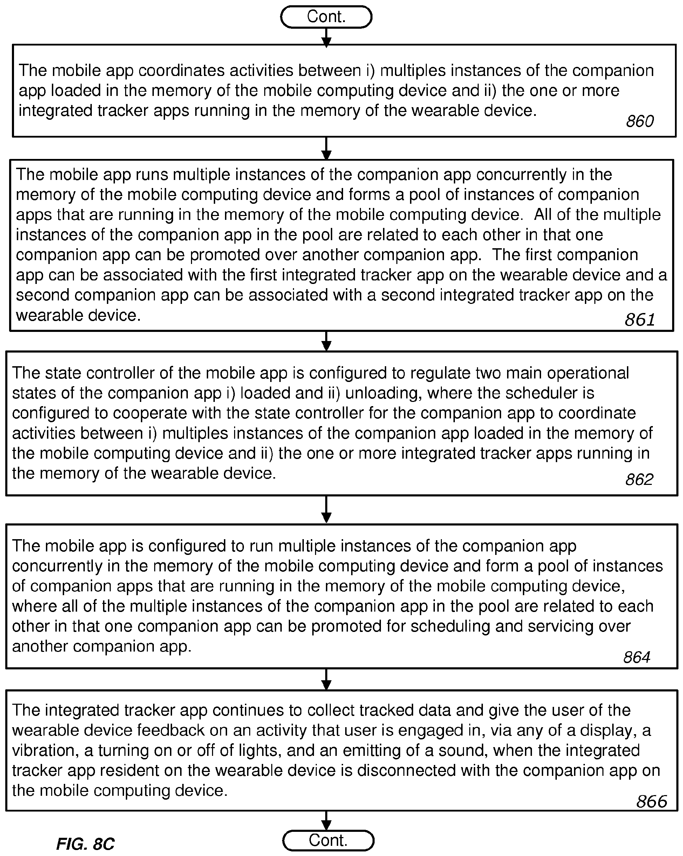

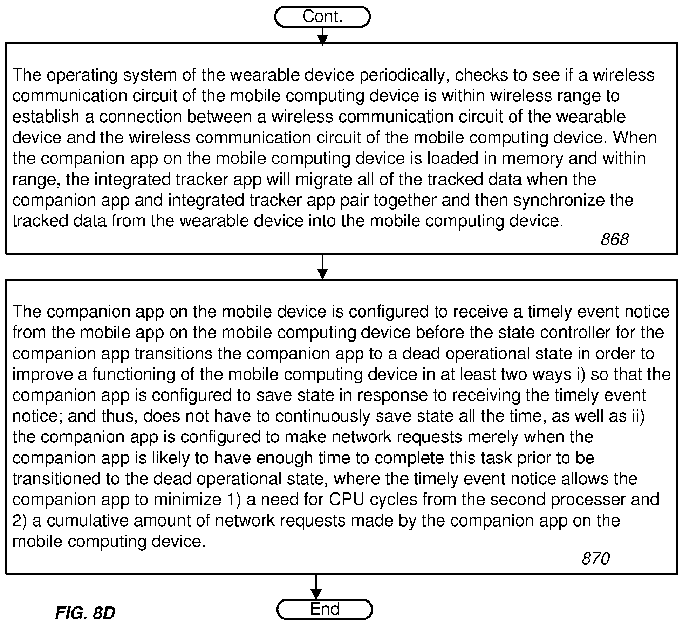

Figures 8A-8D illustrate a method 800 with respect to a mobile app generating and managing one or more companion apps that cooperate with one or more integrated tracker apps to provide a combined functionality to the wearable device in accordance with some embodiments. The method and the steps thereof can be performed out of literal order when logically possible. Data and routines of the methods can be stored on a memory of the wearable electronic device 200, a memory of the mobile computing device 100, and in a combination thereof. The steps of the methods can be executed on the wearable electronic device 200, the mobile computing device 100, or any combination thereof when logically possible.

In step 842, the one or more companion apps in the memory of the mobile computing device cooperate with one or more integrated tracker apps in a memory of a wearable device to provide a combined functionality to the wearable device.

In step 844, the companion app is in communication with an integrated tracker app to provide at least extended capabilities for the integrated tracker app including adding connectivity to an Internet to access On-line services and gather data on behalf of the integrated tracker app via the integrated tracking app submitting a request to a fetch application programming interface for the companion app. The companion app and integrated tracker app may also provide additional functionality of i) performing other data collection by the companion app on behalf of the integrated tracker app, ii) facilitate settings updates for the integrated tracker app on the wearable device configured through a user interface of the companion app displayed on a display screen of the mobile computing device, and iii) extend capabilities of the integrated tracker app via other mechanisms including performing a distributed workflow between the integrated tracker app and the companion app, as well as, obtain sensor data from one or more sensors in the mobile computing device on behalf of the integrated tracker app.

In step 846, the user can configure settings' updates for the integrated tracker app on the wearable device i) through a user interface of the companion app displayed on a display screen of the mobile computing device and ii) through use of a diversity of user input mechanisms of the mobile computing device, including any of a combination of a keyboard input, a voice recognition input, or a touch screen input for the mobile computing device, which allows a user of the integrated tracker app on the wearable device to take advantage of a bigger display screen and a diverse amount of user input mechanisms of the mobile computing device, via the user interface of the companion app, to make settings changes that are then conveyed and implemented on the integrated tracker app on the wearable device.

In step 848, the companion app is run in the memory of the mobile computing device while the wearable device is not running an executable file of the integrated tracker app in order to allow the companion app to extend capabilities of the integrated tracker app by interacting with the Internet web services on behalf of the integrated tracker app while the wearable device is not running the integrated tracker app.

In step 850, the companion app is configured to cooperate with and leverage an authorization proxy for an existing web service to obtain an authentication token to access the user of the wearable device's protected resources at a third party service without asking the user of the wearable device to type their login or password on the wearable device.

In step 852, each integrated tracker app at least consume less battery power and consumes less computing cycles from the first processor on the wearable device than if that integrated tracker apps performed all of the combined functionality given to the wearable device from that integrated tracker app cooperating with the companion app.

In step 854, the integrated tracker app utilizes the mobile computing device's capabilities via the companion app as opposed to interacting directly with an operating system on the mobile computing device.

In step 855, the instructions for the one or more integrated tracker apps are executed by a first processor in the wearable device and the instructions for the one or more companion apps are executed by a second processor in the mobile computing device.

In step 856, the companion app is configured to trigger an operational state of launched in the memory on the mobile computing device for various detected situations of:
i) in response a user launching the integrated tracker app on the wearable device, then a first message is communicated to the state controller to launch the companion app,
ii) when the companion app has requested to be woken up at periodic time intervals because the tracker app and companion have not been in communication for a threshold amount of time, then the companion app is launched in order to pre-fetch data that will be used by the tracker app once it is launched on the wearable device, as well as
iii) when the monitoring routine of the companion app communicates the second message that a significant location change has happened for the wearable device, where the significant location change is equal to or above a location change threshold set by the companion app.

The state controller is configured to transition the companion app into and out of sub states of the main operational states of loaded and unloading in the memory.

In step 860, the mobile app coordinates activities between i) multiples instances of the companion app loaded in the memory of the mobile computing device and ii) the one or more integrated tracker apps running in the memory of the wearable device.

In step 861, the mobile app runs multiple instances of the companion app concurrently in the memory of the mobile computing device and forms a pool of instances of companion apps that are running in the memory of the mobile computing device. All of the multiple instances of the companion app in the pool are related to each other in that one companion app can be promoted over another companion app. The first companion app can be associated with the first integrated tracker app on the wearable device and a second companion app can be associated with a second integrated tracker app on the wearable device.

In step 862, the state controller of the mobile app is configured to regulate two main operational states of the companion app i) loaded and ii) unloading, where the scheduler is configured to cooperate with the state controller for the companion app to coordinate activities between i) multiples instances of the companion app loaded in the memory of the mobile computing device and ii) the one or more integrated tracker apps running in the memory of the wearable device.

In step 864, the mobile app is configured to run multiple instances of the companion app concurrently in the memory of the mobile computing device and form a pool of instances of companion apps that are running in the memory of the mobile computing device, where all of the multiple instances of the companion app in the pool are related to each other in that one companion app can be promoted for scheduling and servicing over another companion app.

In step 866, the integrated tracker app continues to collect tracked data and give the user of the wearable device feedback on an activity that user is engaged in, via any of a display, a vibration, a turning on or off of lights, and an emitting of a sound, when the integrated tracker app resident on the wearable device is disconnected with the companion app on the mobile computing device.

In step 868, the operating system of the wearable device periodically, checks to see if a wireless communication circuit of the mobile computing device is within wireless range to establish a connection between a wireless communication circuit of the wearable device and the wireless communication circuit of the mobile computing device. When the companion app on the mobile computing device is loaded in memory and within range, the integrated tracker app will migrate all of the tracked data when the companion app and integrated tracker app pair together and then synchronize the tracked data from the wearable device into the mobile computing device.

In step 870, the companion app on the mobile device is configured to receive a timely event notice from the mobile app on the mobile computing device before the state controller for the companion app transitions the companion app to a dead operational state in order to improve a functioning of the mobile computing device in at least two ways i) so that the companion app is configured to save state in response to receiving the timely event notice; and thus, does not have to continuously save state all the time, as well as ii) the companion app is configured to make network requests merely when the companion app is likely to have enough time to complete this task prior to be transitioned to the dead operational state, where the timely event notice allows the companion app to minimize 1) a need for CPU cycles from the second processer and 2) a cumulative amount of network requests made by the companion app on the mobile computing device.

In some embodiments, the software used to facilitate the algorithms discussed herein can be embodied onto a non-transitory machine-readable medium. A machine-readable medium includes any mechanism that stores information in a form readable by a machine (e.g., a computer). For example, a non-transitory machine-readable medium can include read only memory (ROM); random access memory (RAM); magnetic disk storage media; optical storage media; flash memory devices; Digital Versatile Disc (DVD's), EPROMs, EEPROMs, FLASH memory, magnetic or optical cards, or any type of media suitable for storing electronic instructions. However, this does not include transitory signals or waves carrying information.

Some portions of the detailed descriptions above are presented in terms of algorithms and symbolic representations of operations on data bits within a computer memory. These algorithmic descriptions and representations are the means used by those skilled in the data processing arts to most effectively convey the substance of their work to others skilled in the art. An algorithm is here, and generally, conceived to be a self-consistent sequence of steps leading to a desired result. The steps are those requiring physical manipulations of physical quantities. Usually, though not necessarily, these quantities take the form of electrical or magnetic signals capable of being stored, transferred, combined, compared, and otherwise manipulated. It has proven convenient at times, principally for reasons of common usage, to refer to these signals as bits, values, elements, symbols, characters, terms, numbers, or the like. These algorithms can be written in a number of different software programming languages such as C, C+, JavaScript, or other similar languages. Also, an algorithm can be implemented with lines of code in software, configured logic gates in software, or a combination of both. In an embodiment, the logic consists of electronic circuits that follow the rules of Boolean Logic, software that contain patterns of instructions, or any combination of both.

It should be borne in mind, however, that all of these and similar terms are to be associated with the appropriate physical quantities and are merely convenient labels applied to these quantities. Unless specifically stated otherwise as apparent from the above discussions, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system's registers and memories into other data similarly represented as physical quantities within the computer system memories or registers, or other such information storage, transmission or display devices.

Although embodiments of this design have been fully described with reference to the accompanying drawings, it is to be noted that various changes and modifications will become apparent to those skilled in the art. For example, most functions performed by electronic hardware components can be duplicated by software emulation, and vice versa. Thus, a software program written to accomplish those same functions can emulate the functionality of the hardware components in input-output circuitry, and vice versa. Such changes and modifications are to be understood as being included within the scope of embodiments of this design as defined by the appended claims. The invention is to be understood as not limited by the specific embodiments described herein, but only by scope of the appended claims.

## Claims

1. A non-transitory machine-readable medium configured to store instructions and data, which when executed by one or more processors on a mobile computing device, causes the following operations, comprising:
a mobile app generating and managing one or more companion apps in a memory of the mobile computing device, where the one or more companion apps in the memory of the mobile computing device cooperate with one or more integrated tracker apps in a memory of a wearable device to provide a combined functionality to the wearable device, where a first companion app is in communication with a first integrated tracker app to provide at least extended capabilities for the first integrated tracker app including adding connectivity to an Internet to access On-line services and gather data on behalf of the first integrated tracker app via the first integrated tracking app submitting a request to a fetch application programming interface for the first companion app;
executing instructions for the first integrated tracker app with a first processor in the wearable device, and executing instructions for the first companion app with a second processor in the mobile computing device;
where the mobile app coordinates activities between i) multiples instances of the companion app loaded in the memory of the mobile computing device and ii) the one or more integrated tracker apps running in the memory of the wearable device;
where the first integrated tracker app at least consumes less battery power and consumes less computing cycles from the first processor on the wearable device than if the first integrated tracker app performed all of the combined functionality given to the wearable device from the first integrated tracker app cooperating with the first companion app; and
where the first integrated tracker app utilizes the mobile computing device's capabilities via the first companion app as opposed to interacting directly with an operating system on the mobile computing device.

2. The non-transitory machine-readable medium of claim 1 storing instructions to further cause the following operations, comprising:
where the mobile app is configured to run multiple instances of the companion app concurrently in the memory of the mobile computing device and form a pool of instances of companion apps that are running in the memory of the mobile computing device, where all of the multiple instances of the companion app in the pool are related to each other in that one companion app can be promoted over another companion app, where the first companion app is associated with the first integrated tracker app on the wearable device, a second companion app is associated with a second integrated tracker app on the wearable device.

3. An apparatus, comprising:
a mobile app resident in a memory of a mobile computing device having multiple components including one or more event handers, a state controller, a scheduler, and an application programming interface; where the mobile app is configured to generate and manage one or more companion apps in the memory of the mobile computing device, where the one or more companion apps in the memory of the mobile computing device are coded to cooperate with one or more integrated tracker apps in a memory of the wearable device to provide a combined functionality to the wearable device;
where a first companion app is coded to be in communication with a first integrated tracker app to provide functionality selected from a group consisting of i) perform data collection by the first companion app on behalf of the first integrated tracker app, ii) facilitate settings updates for the first integrated tracker app on the wearable device configured through a user interface of the first companion app displayed on a display screen of the mobile computing device, and iii) extend capabilities of the first integrated tracker app;
where the one or more integrated tracker apps are resident in the memory of the wearable device and are configured to wirelessly pair through a wireless communication circuit of the wearable device to the one or more companion apps loaded in the memory of the mobile computing device, where the first integrated tracker app on the wearable device is configured to submit requests for the one or more event handlers through the application programming interface of the mobile app, where the instructions for the first integrated tracker app are executed by a first processor of the wearable device and instructions for the first companion app are executed by a second processor of the mobile computing device;
where the first companion app is configured to run in the memory of the mobile computing device while the wearable device is not running an executable file of the first integrated tracker app in order to allow the first companion app to perform functions selected from a group consisting of i) pre-fetch data on behalf of the first integrated tracker app, ii) record settings updates to the first integrated tracker app on the wearable device that are configured through the user interface of the companion app displayed on the display screen of the mobile computing device, iii) extend a computational and memory storage capabilities available to the first integrated tracker app via a distributed workflow between the first integrated tracker app and the companion app, iv) extend capabilities of the first integrated tracker app by adding connectivity to an Internet to access On-line services and gather data on behalf of the first integrated tracker app via the first integrated tracking app submitting a request to the application programming interface for the first companion app, and v) obtain sensor data from one or more sensors in the mobile computing device on behalf of the first integrated tracker app;
wherein the state controller of the mobile app is configured to regulate two main operational states of the first companion app i) loaded and ii) unloading, where the scheduler is configured to cooperate with the state controller for the companion app to coordinate activities between i) multiples instances of the companion app loaded in the memory of the mobile computing device and ii) the one or more integrated tracker apps running in the memory of the wearable device; and
where the first integrated tracker app occupies less memory space, consumes less battery power, and consumes less computing cycles from the first processor on the wearable device than if the first integrated tracker app performed all of the combined functionality given to the wearable device from the first integrated tracker app cooperating with the first companion app.

4. The apparatus of claim 3, where the state controller for the companion app is configured to trigger an operational state of launched in the memory on the mobile computing device for various detected situations selected from a group consisting of
i) in response a user launching the first integrated tracker app on the wearable device, then a first message is communicated to the state controller to launch the first companion app,
ii) when the first companion app has requested to be woken up at periodic time intervals because the first integrated tracker app and first companion have not been in communication for a threshold amount of time, then the first companion app is launched in order to pre-fetch data that will be used by the first tracker app once it is launched on the wearable device, as well as
iii) when the monitoring routine of the companion app communicates the second message that a significant location change has happened for the wearable device, where the significant location change is equal to or above a location change threshold set by the companion app.

5. The apparatus of claim 4, where the first companion app is configured to provide integrated functionality on behalf of the first integrated app for all three of i) data collect by the first companion app on behalf of the first integrated tracker app, ii) provide settings updates for the first integrated tracker app on the wearable device configured through the user interface of the first companion app displayed on the display screen of the mobile computing device, as well as iii) extend capabilities of the first integrated tracker app;
where the state controller for the companion app is configured to trigger an operational state of launched in the memory on the mobile computing device for all three of
i) in response the user launching the first integrated tracker app on the wearable device, then the first message is communicated to the state controller to launch the first companion app,
ii) when the first companion app has requested to be woken up at periodic time intervals because the tracker app and companion have not been in communication for a threshold amount of time, then the first companion app is launched in order to pre-fetch data that will be used by the first tracker app once it is launched on the wearable device, as well as
iii) when the monitoring routine of the companion app communicates the second message that the significant location change has happened for the wearable device, where the significant location change is equal to or above the location change threshold set by the companion app; and
where the mobile app is not part of an operating system of the mobile computing device but rather a standalone mobile app; where the wearable device is a fitness tracker and the mobile computing device is a smart phone.

6. The apparatus of any of claims 3 to 5, where the state controller is configured to transition the first companion app into and out of sub states of the main operational state of unloading in the memory, where the sub states include at least i) Dead - awaiting launch of the first companion app via the state controller; and ii) Dying - the state controller is coded to expect a communication from the first integrated tracker app that the first integrated tracker app will not send any more events during this life cycle of the first companion app and accordingly the first companion app should be unloaded from the memory of the mobile computing device when all of the event handlers for the first companion app return their results back to the first integrated tracker app.

7. The apparatus of any of claims 3 to 6, where the state controller is configured to transition the first companion app into and out of sub states of the main operational state of loaded in the memory, where the sub states include at least i) Queued - the state controller has triggered the first companion app to be executed but it is awaiting an available slot by the scheduler; ii) Launched - the first companion app is being executed and run in the memory of the mobile computing device, iii) Promoted - the first companion app is running concurrently with other companion apps and has priority for scheduling and servicing over one or more of the companion apps, and in addition the first companion app will keep this sub state context running until an event triggers its demotion, iv) Demoted - the first companion app is handling events but no corresponding first integrated tracker app is currently running in the memory of wearable device and, optionally,
where the state controller is configured to trigger the sub state of launched for the first companion app on the mobile computing device when the first tracker app has been launched on the wearable device by the user so that the first companion app can perform any of i) pre-fetch data from an Internet source and ii) pre-fetch sensor data from sensors on the mobile computing device; and thereby, minimize a time period it takes for the first tracker app to display up to date information on a display screen of the wearable device when the user launches the first tracker app on the wearable device.

8. The apparatus of any of claims 3 to 7, where the scheduler of the mobile app is configured to run multiple instances of the companion app concurrently in the memory of the mobile computing device and form a pool of instances of companion apps that are running in the memory of the mobile computing device, where all of the multiple instances of the companion app in the pool are related to each other in that one companion app can be promoted for scheduling and servicing over another companion app, or
where the scheduler of the mobile app is configured to limit an amount of instances of companion apps running concurrently in the pool to a threshold set amount, where when launching another instance of the companion app would violate the threshold set amount of the pool, then the scheduler will put a next instance of the companion app into a priority queue, where in the priority queue, promoted companion apps have priority over all other companion apps in a different sub state including a demoted companion app.

9. The apparatus of any of claims 3 to 8, where the first companion app is configured to cooperate with and leverage an authorization proxy for an existing web service to obtain an authentication token to access the user of the wearable device's protected resources at a third party service without asking the user of the wearable device to type their login or password on the wearable device.

10. The apparatus of any of claims 3 to 9,
where the first integrated tracker app resident on the wearable device is configured to collect the user's tracked data, where the first integrated tracker app is coded that when disconnected with the first companion app on the mobile computing device, then the first integrated tracker app is configured to continue to collect information and give the user of the wearable device feedback on an activity that user is engaged in, via any of a display, a vibration, a turning on or off of lights, and an emitting of a sound,
where a tracker operating system of the wearable device is configured to periodically check to see if the wireless communication circuit of the mobile computing device is within wireless range to establish a connection between the wireless communication circuit of the wearable device and the wireless communication circuit of the mobile computing device, and
where when the first companion app on the mobile computing device is loaded in the memory and within range to establish the connection, the first integrated tracker app is configured to migrate all of the tracked data when the two applications communicate with each other, and then the first companion app is configured to synchronize the tracked data from the wearable device into the mobile computing device.

11. The apparatus of any of claims 3 to 10, where the first companion app on the mobile device is configured to receive a timely event notice from the mobile app on the mobile computing device before the state controller for the first companion app transitions the first companion app to a dead operational state in order to improve a functioning of the mobile computing device in at least two ways i) so that the first companion app is configured to save state in response to receiving the timely event notice; and thus, does not have to continuously save state all the time, as well as ii) the first companion app is configured to make network requests merely when the first companion app is likely to have enough time to complete this task prior to be transitioned to the dead operational state, where the timely event notice allows the first companion app to minimize 1) a need for CPU cycles from the second processer and 2) a cumulative amount of network requests made by the first companion app on the mobile computing device.

12. A method for one or more tracker apps in a memory of wearable device to cooperate with one or more companion apps in a memory of a mobile computing device, comprising:
a mobile app generating and managing the one or more companion apps in the memory of the mobile computing device, where the one or more companion apps in the memory of the mobile computing device cooperate with the one or more integrated tracker apps in the memory of the wearable device to provide a combined functionality to the wearable device, where a first companion app is in communication with a first integrated tracker app to provide at least extended capabilities for the first integrated tracker app including adding connectivity to an Internet to access On-line services and gather data on behalf of the first integrated tracker app via the first integrated tracking app submitting a request to a fetch application programming interface for the first companion app;
executing instructions for the first integrated tracker apps with a first processor in the wearable device, and executing instructions for the first companion app with a second processor in the mobile computing device;
where the mobile app coordinates activities between i) multiples instances of the companion app loaded in the memory of the mobile computing device and ii) the one or more integrated tracker apps running in the memory of the wearable device;
where the first integrated tracker app at least consumes less battery power and consumes less computing cycles from the first processor on the wearable device than if the first integrated tracker app performed all of the combined functionality given to the wearable device from the first integrated tracker app cooperating with the first companion app; and
where the first integrated tracker app utilizes the mobile computing device's capabilities via the first companion app as opposed to interacting directly with an operating system on the mobile computing device.

13. The non-transitory machine-readable medium of claim 1 or claim 2 storing instructions to further cause the following operations, or the method of claim 12, further comprising:
triggering the companion app to launch in the memory on the mobile computing device in response a user launching the first integrated tracker app on the wearable device;
running the first companion app in the memory of the mobile computing device while the wearable device is not running an executable file of the first integrated tracker app in order to allow the companion app to extend capabilities of the first integrated tracker app by interacting with the Internet web services on behalf of the first integrated tracker app while the wearable device is not running the first integrated tracker app; and
further extending a computational and memory storage capabilities available to the first integrated tracker app by the first companion app performing a distributed workflow using the second processor and memory of the mobile computing device on behalf of the first integrated tracker app.

14. The non-transitory machine-readable medium of claim 1, claim 2 or claim 13, or the method of claim 12 or claim 13, further comprising:
continuing to collect tracked data and give the user of the wearable device feedback on an activity that user is engaged in, via any of a display, a vibration, a turning on or off of lights, and an emitting of a sound, when the first integrated tracker app resident on the wearable device is disconnected with the first companion app on the mobile computing device; and
periodically, checking to see if a wireless communication circuit of the mobile computing device is within wireless range to establish a connection between a wireless communication circuit of the wearable device and the wireless communication circuit of the mobile computing device, where when the first companion app on the mobile computing device is loaded in memory and within range, the first integrated tracker app will migrate all of the tracked data when the companion app and first integrated tracker app pair together and then synchronize the tracked data from the wearable device into the mobile computing device.

15. The non-transitory machine-readable medium of claim 1, claim 2, claim 13 or claim 14, or the method of any of claims 12 to 14, further comprising:
configuring settings' updates for the first integrated tracker app on the wearable device i) through a user interface of the first companion app displayed on a display screen of the mobile computing device and ii) through use of a diversity of user input mechanisms of the mobile computing device, including any of a combination of a keyboard input, a voice recognition input, or a touch screen input for the mobile computing device, which allows a user of the tracker app on the wearable device to take advantage of a bigger display screen and diverse user input mechanisms of the mobile computing device, via the user interface of the first companion app, to make setting changes that are then conveyed and implemented on the first tracker app on the wearable device.
